# EUROPEAN PATENT APPLICATION

(11) **EP 4 617 258 A1**
(43) Date of publication of application: **17.09.2025**
(21) Application number: 23888884.6
(22) Date of filing: 31.08.2023
(51) Int. Cl.: C07C 219/18, C07C 213/02, C07C 213/08

(54) **CATIONIC LIPIDS AND MANUFACTURING METHOD THEREFOR**

(30) Priority: 08.11.2022 KR 20220148222
(71) Applicant: Samyang Holdings Corporation, Seoul 03129 (KR)
(72) Inventor: PARK, Jong Min, Seongnam-si, Gyeonggi-do 13590 (KR); LEE, So Jin, Seoul 02468 (KR); SEONG, Shi Hwa, Seoul 06008 (KR); KYUNG, Kyu Jin, Yongin-si, Gyeonggi-do 16827 (KR); KIM, Sol, Seongnam-si, Gyeonggi-do 13488 (KR); NAM, Joung Pyo, Seoul 02471 (KR); YOON, Yoo Jeong, Seongnam-si, Gyeonggi-do 13544 (KR)
(74) Representative: Gleiss Große Schrell und Partner mbB
(86) International application number: PCT/KR2023/013008
(87) International publication number: WO 2024/101618

(57) **Abstract**

The present invention relates to cationic lipids and a manufacturing method therefor. More specifically, the present invention is concerned with cationic lipids that can easily form complexes with anionic drugs and thus are useful for drug delivery, and a method for manufacturing same.

## Description

### TECHNICAL FIELD

The present invention relates to a cationic lipid and a method for preparing the same, and more specifically, the present invention relates to a cationic lipid which can easily form a complex with anionic drug and thus is useful for drug delivery, and a method for preparing the same.

### BACKGROUND ART

In therapies using anionic drugs including nucleic acid, technologies for safe and efficient drug delivery have been researched for a long time, and various carriers and techniques for delivery have been developed. Carriers are mainly divided into viral carriers utilizing adenovirus, retrovirus or the like, and non-viral carriers utilizing cationic lipid, cationic polymer or the like. Viral carriers are known as being exposed to risks such as non-specific immune response, etc. and having many problems in commercialization due to the complexity of the production process. Thus, recent researches proceed in the direction to improve such disadvantages by using non-viral carriers. In comparison with viral carriers, non-viral carriers have advantages of fewer side effects in terms of *in vivo* safety, and lower production cost in terms of economy.

The representative non-viral carriers for delivering nucleic acid material are a complex of cationic lipid and nucleic acid (lipoplex) and a complex of polycationic polymer and nucleic acid (polyplex). Such a cationic lipid or polycationic polymer stabilizes anionic drug by forming a complex through electrostatic interaction with the anionic drug and increases intracellular delivery, and for these reasons, various researches thereof have been conducted (De Paula D, Bentley MV, Mahato RI, Hydrophobization and bioconjugation for enhanced siRNA delivery and targeting, RNA 13 (2007) 431-56; Gary DJ, Puri N, Won YY, Polymer-based siRNA delivery: Perspectives on the fundamental and phenomenological distinctions from polymer-based DNA delivery, J Control release 121 (2007) 64-73).

However, polycation polymers have cytotoxicity due to their multivalent cationic charges, making them problematic for practical use, and conventional cationic lipids used in ionic lipid nanoparticles including cationic lipid, neutral lipid and fusogenic lipid have the disadvantages of complication in the synthesis method, cytotoxicity, and low efficiency of intracellular nucleic acid delivery.

### CONTENTS OF THE INVENTION

### PROBLEMS TO BE SOLVED

The purpose of the present invention is to provide a cationic lipid having a specific structure which can easily form a complex with anionic drug and thus is useful for drug delivery, and a method for preparing the same.

### TECHNICAL MEANS

The first aspect of the present invention provides a lipid having a structure represented by the following formula (1): 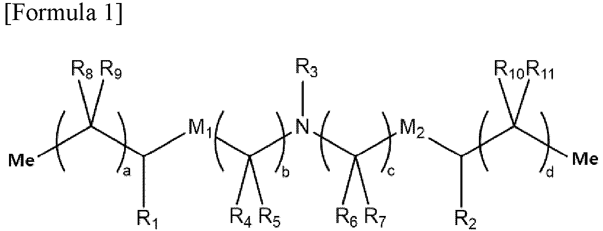 wherein, in the above formula (1),
each of M₁ and M₂ is independently a divalent linker group,
each of R₁ and R₂ is independently a substituted or unsubstituted carbocyclic group or heterocyclic group,
R₃ is hydrogen atom, or a substituted or unsubstituted organic group optionally comprising one or more heteroatoms,
each of R₄ to R₁₁ is independently hydrogen atom, or a substituted or unsubstituted, saturated or unsaturated hydrocarbon group,
Me is methyl group, and
each of a, b, c and d is independently an integer of from 1 to 20.

According to an embodiment of the present invention, in the above formula (1), each of M₁ and M₂ may be independently selected from the group consisting of -C(O)O-, -OC(O)-, - OC(O)-M'-C(O)O-, -C(O)N(R')-, -N(R')C(O)-, -C(O)-, -C(S)-, -C(S)S-, -SC(S)-, -CH(OH)-, - P(O)(OR')O-, -S(O)₂-, -S-S-, arylene (more concretely, C₆₋₂₀ arylene), and heteroarylene (more concretely, C₃₋₂₀ heteroarylene having one or more (e.g., 1 to 3) heteroatoms selected from N, O and S), wherein M' may be a direct bond, C₁₋₁₃ alkylene or C₂₋₁₃ alkenylene, and each R' may be independently selected from the group consisting of hydrogen atom, C₁₋₁₈ alkyl and C₂₋₁₈ alkenyl.

According to an embodiment of the present invention, in the above formula (1), each of R₁ and R₂ may be independently selected from the group consisting of substituted or unsubstituted C₃₋₂₀ cycloalkyl, substituted or unsubstituted C₃₋₂₀ cycloalkenyl, substituted or unsubstituted C₆₋₂₀ aryl, substituted or unsubstituted C₃₋₂₀ heterocycloalkyl, substituted or unsubstituted C₃₋₂₀ heterocycloalkenyl, and substituted or unsubstituted C₃₋₂₀ heteroaryl, wherein each of the heterocycloalkyl, heterocycloalkenyl and heteroaryl may independently have one or more (e.g., 1 to 3) heteroatoms selected from N, O and S.

According to an embodiment of the present invention, in the above formula (1), R₃ may be selected from the group consisting of hydrogen atom, substituted or unsubstituted C₁₋₆ alkyl, substituted or unsubstituted C₃₋₆ carbocyclic group, -(CH₂)ₙQ, - (CH₂)ₙCHQR, -CHQR and - CQ(R)₂, wherein each R may be independently selected from the group consisting of hydrogen atom, C₁₋₃ alkyl and C₂₋₃ alkenyl; Q may be selected from the group consisting of carbocyclic group, heterocyclic group, -OR, -O(CH₂)ₙN(R)₂, -C(O)OR, -OC(O)R, -CX₃, -CX₂H, -CXH₂, -CN, -N(R)₂, -C(O)N(R)₂, -N(R)C(O)R, -N(R)S(O)₂R, -N(R)C(O)N(R)₂, -N(R)C(S)N(R)₂, -N(R)R₁₂, N(R)S(O)2R₁₂, -O(CH2)ₙOR, -N(R)C(=NR₁₃)N(R)₂, -N(R)C(=CHR₁₃)N(R)₂, -OC(O)N(R)₂, - N(R)C(O)OR, -N(OR)C(O)R, -N(OR)S(O)₂R, -N(OR)C(O)OR, -N(OR)C(O)N(R)₂, - N(OR)C(S)N(R)₂, -N(OR)C(=NR₁₃)N(R)₂, -N(OR)C(=CHR₁₃)N(R)₂, -C(=NR₁₃)N(R)₂, - C(=NR₁₃)R, -C(O)N(R)OR and -C(R)N(R)₂C(O)OR, wherein each n is independently an integer of from 1 to 5; R₁₂ is selected from the group consisting of C₃₋₆ carbocyclic group and heterocyclic group; R₁₃ is selected from the group consisting of H, CN, NO₂, C₁₋₆ alkyl, -OR, -S(O)₂R, - S(O)₂N(R)₂, C₂₋₆ alkenyl, C₃₋₆ carbocyclic group and heterocyclic group; each R is independently selected from the group consisting of hydrogen atom, C₁₋₃ alkyl and C₂₋₃ alkenyl; each X is independently selected from the group consisting of F, CI, Br and I, provided that when R₃ is - (CH₂)ₙQ, -(CH₂)ₙCHQR, -CHQR or -CQ(R)₂, (i) if n is 1, 2, 3, 4, or 5, then Q is not -N(R)₂, or (ii) if n is 1 or 2, Q is not 5-, 6- or 7-membered heterocycloalkyl.

According to an embodiment of the present invention, in the above formula (1), each of R₄ to R₁₁ may be independently selected from the group consisting of hydrogen atom, C₁₋₃ alkyl and C₂₋₃ alkenyl.

According to an embodiment of the present invention, in the above formula (1), each of a, b, c and d may be independently an integer of from 1 to 15.

More concretely, in the above formula (1), each of M₁ and M₂ may be independently selected from the group consisting of -C(O)O-, -OC(O)-, -OC(O)-M'-C(O)O-, -C(O)N(R')-, - N(R')C(O)- and -C(O)- (wherein M' and R' are the same as defined above).

More concretely, in the above formula (1), each of R₁ and R₂ may be independently selected from the group consisting of substituted or unsubstituted C₃₋₂₀ cycloalkyl and substituted or unsubstituted C₃₋₂₀ heterocycloalkyl, wherein the heterocycloalkyl may have one or more (e.g., 1 to 3) heteroatoms selected from N, O and S.

More concretely, in the above formula (1), R₃ may be selected from the group consisting of hydrogen atom, substituted or unsubstituted C₁₋₆ alkyl, and substituted or unsubstituted C₃₋₆ carbocyclic group (e.g., C₃₋₆ cycloalkyl).

More concretely, in the above formula (1), each of R₄ to R₁₁ may be independently hydrogen atom or C₁₋₃ alkyl.

More concretely, in the above formula (1), each of a, b, c and d may be independently an integer of from 3 to 11.

Still more concretely, in the above formula (1), each of M₁ and M₂ may be independently -C(O)O- or -OC(O)-.

Still more concretely, in the above formula (1), each of R₁ and R₂ may be independently substituted or unsubstituted C₃₋₆ cycloalkyl.

Still more concretely, in the above formula (1), R₃ may be hydrogen atom or unsubstituted C₁₋₃ alkyl.

Still more concretely, in the above formula (1), R₄ to R₁₁ may be hydrogen atom.

Still more concretely, in the above formula (1), each of a, b, c and d may be independently an integer of from 5 to 9.

Even more concretely, the lipid may be one having a structure selected from the following formulas A to O:

| Formula | Structure |
|---|---|
| A | 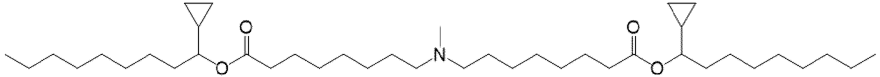 |
| B | 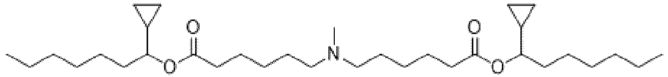 |
| C | 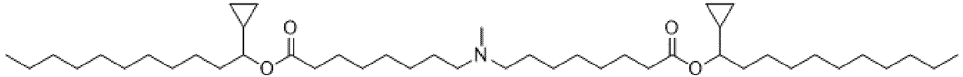 |
| D | 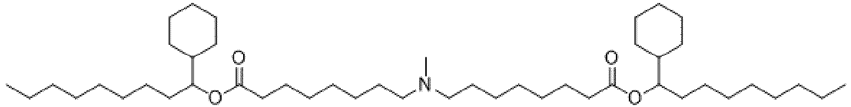 |
| E | 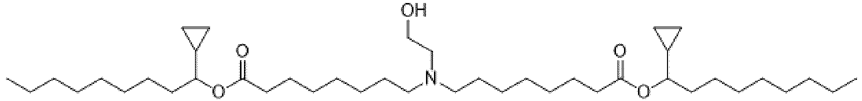 |
| F | 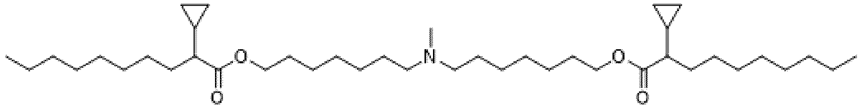 |
| G | 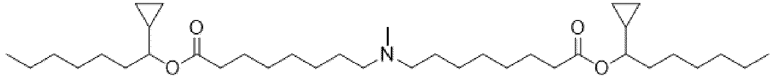 |
| H | 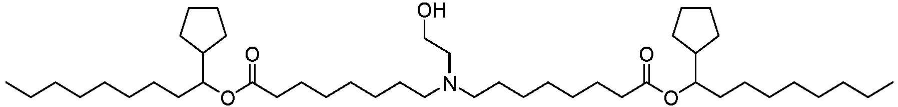 |
| I | 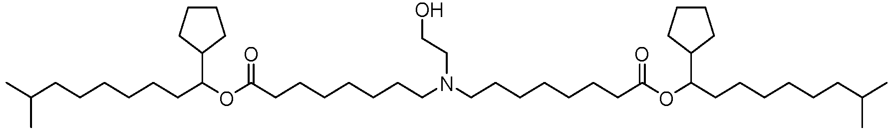 |
| J | 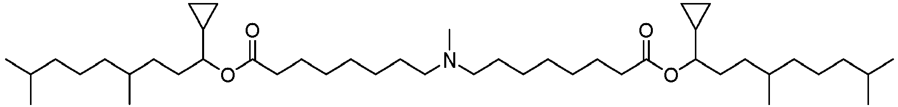 |
| K | 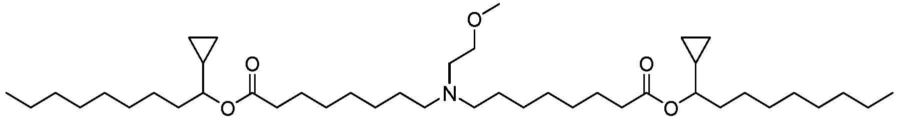 |
| L | 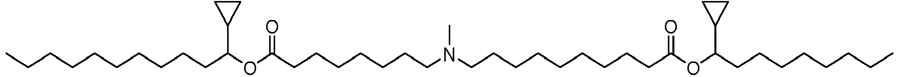 |
| M | 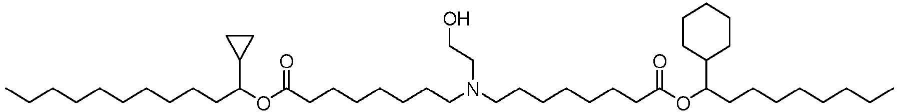 |
| N | 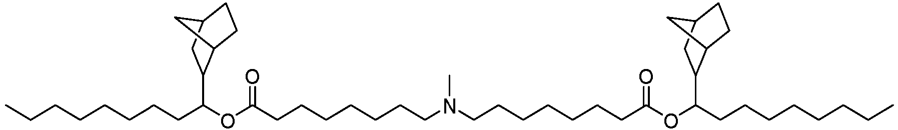 |
| O | 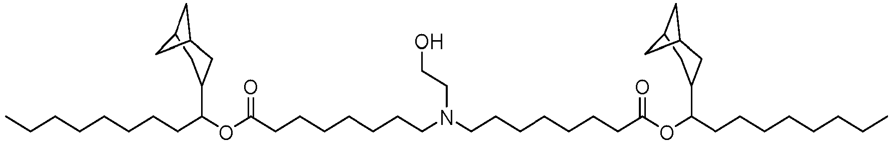 |

The second aspect of the present invention provides a method for preparing a lipid having a structure represented by formula (1'), comprising steps of: (1) reacting a compound of formula (a) with a compound of formula (b) to obtain a compound of formula (c); (2) reacting a compound of formula (c) with a compound of formula (d) to obtain a compound of formula (e); and (3) reacting a compound of formula (e) with a compound of formula (f):

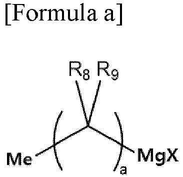

[Formula b] R₁-CHO

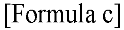

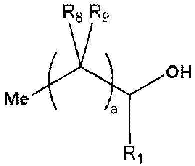

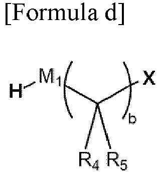

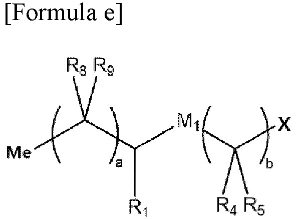

[Formula f] R₃-NH;

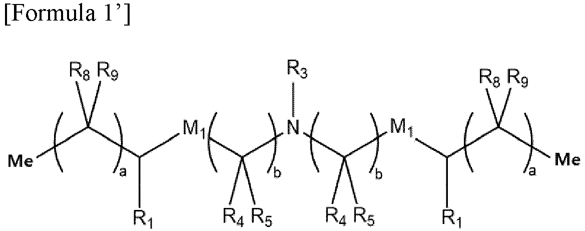

wherein,
M₁, R₁, R₃, R₄, R₅, R₈, R₉, Me, a and b are the same as defined in formula (1) above, and
each X is independently selected from the group consisting of F, CI, Br and I.

The third aspect of the present invention provides a method for preparing a lipid having a structure represented by formula (1), comprising steps of: (1) reacting a compound of formula (a') with a compound of formula (b') to obtain a compound of formula (c'); (2) reacting a compound of formula (c') with a compound of formula (d') to obtain a compound of formula (e'); and (3) reacting a compound of formula (e') and a compound of formula (e) obtained from the second aspect of the present invention with a compound of formula (f):

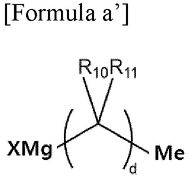

[Formula b'] R₂-CHO

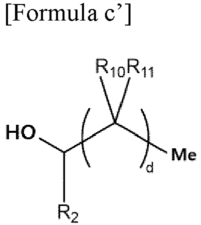

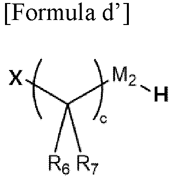

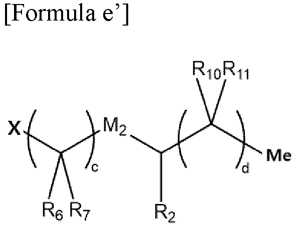

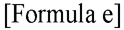

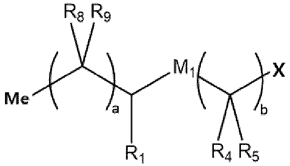

[Formula f] R₃-NH2

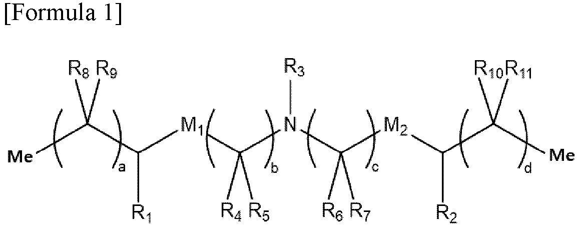

wherein,
M₁, M₂, R₁ to R₁₁, Me, a, b, c and d are the same as defined in formula (1) above, and
each X is independently selected from the group consisting of F, CI, Br and I.

The fourth aspect of the present invention provides a method for preparing a lipid having a structure represented by formula (1'), comprising steps of: (1) reacting a compound of formula (i) with a compound of formula (ii) to obtain a compound of formula (iii); (2) reacting a compound of formula (iii) with a compound of formula (iv) to obtain a compound of formula (v); (3) reacting a compound of formula (v) with a compound of formula (vi) to obtain a compound of formula (vii); and (4) reacting a compound of formula (v) with a compound of formula (vii):

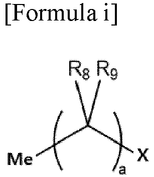

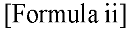

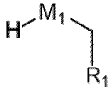

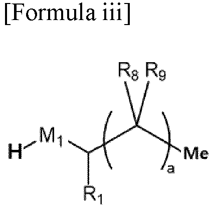

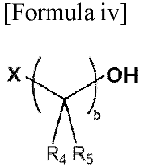

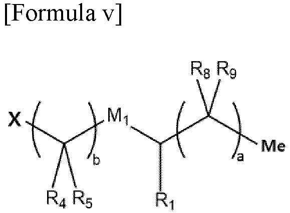

[Formula vi] R₃-NH;

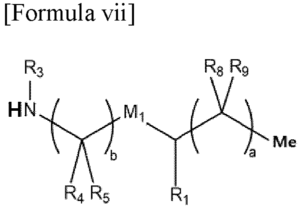

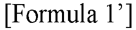

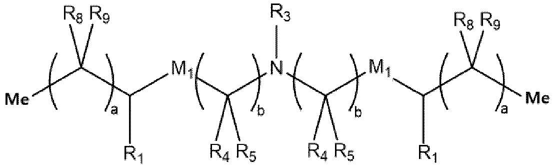

wherein,
M₁, R₁, R₃, R₄, R₅, R₈, R₉, Me, a and b are the same as defined in formula (1) above, and
each X is independently selected from the group consisting of F, CI, Br and I.

The fifth aspect of the present invention provides a method for preparing a lipid having a structure represented by formula (1), comprising steps of: (1) reacting a compound of formula (i') with a compound of formula (ii') to obtain a compound of formula (iii'); (2) reacting a compound of formula (iii') with a compound of formula (iv') to obtain a compound of formula (v'); (3) reacting a compound of formula (v') with a compound of formula (vi) to obtain a compound of formula (vii'); and (4) reacting a compound of formula (vii') with a compound of formula (v) obtained from the fourth aspect of the present invention:

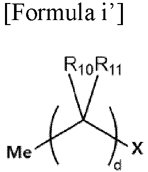

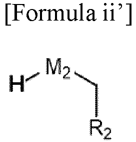

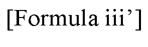

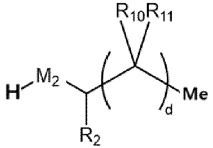

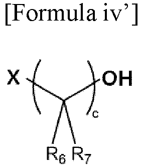

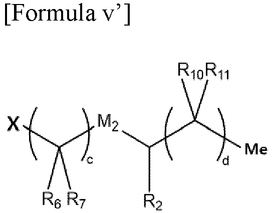

[Formula vi] R₃-NH;

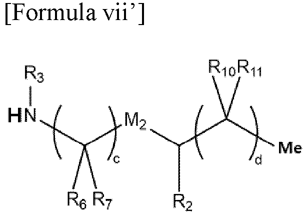

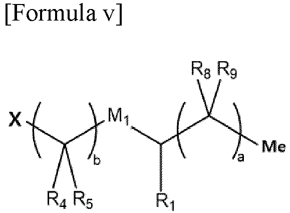

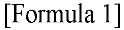

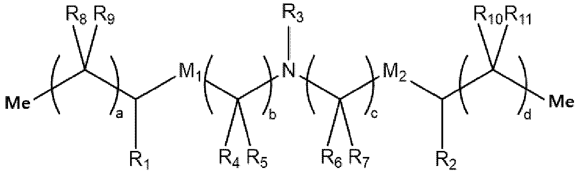

wherein,
M₁, M₂, R₁ to R₁₁, Me, a, b, c and d are the same as defined in formula (1) above, and
each X is independently selected from the group consisting of F, CI, Br and I.

The sixth aspect of the present invention provides a composition for drug delivery comprising the lipid according to the present invention.

### EFFECT OF THE INVENTION

The lipid having a specific structure according to the present invention can easily form a complex with anionic drug, and by utilizing the complex, the drug can be efficiently delivered into the targeted living tissue.

### BRIEF EXPLANATION OF THE DRAWINGS

Figure 1 is a reaction scheme for the lipid synthesis procedure conducted in Example 1.
Figure 2 is a reaction scheme for the lipid synthesis procedure conducted in Example 2.
Figure 3 is a reaction scheme for the lipid synthesis procedure conducted in Example 3.
Figure 4 is a reaction scheme for the lipid synthesis procedure conducted in Example 4.
Figure 5 is a reaction scheme for the lipid synthesis procedure conducted in Example 5.
Figure 6 is a reaction scheme for the lipid synthesis procedure conducted in Example 6.
Figure 7 is a reaction scheme for the lipid synthesis procedure conducted in Example 7.
Figure 8 is a reaction scheme for the lipid synthesis procedure conducted in Example 8.
Figure 9 is a reaction scheme for the lipid synthesis procedure conducted in Example 9.
Figure 10 is a reaction scheme for the lipid synthesis procedure conducted in Example 10.
Figure 11 is a reaction scheme for the lipid synthesis procedure conducted in Example 11.
Figure 12 is a reaction scheme for the lipid synthesis procedure conducted in Example 12.
Figure 13 is a reaction scheme for the lipid synthesis procedure conducted in Example 13.
Figure 14 is a reaction scheme for the lipid synthesis procedure conducted in Example 14.
Figure 15 is a reaction scheme for the lipid synthesis procedure conducted in Example 15.

### CONCRETE MODE FOR CARRYING OUT THE INVENTION

The present invention will be explained in detail below.

A lipid provided by the first aspect of the present invention has a structure represented by the following formula (1): 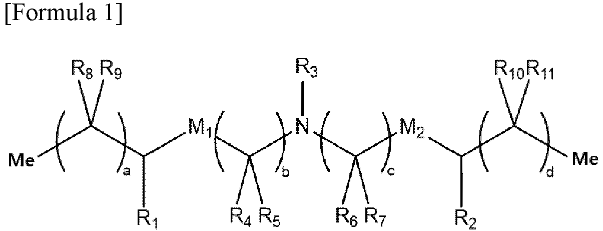 wherein, in the above formula (1),
each of M₁ and M₂ is independently a divalent linker group,
each of R₁ and R₂ is independently a substituted or unsubstituted carbocyclic group or heterocyclic group,
R₃ is hydrogen atom, or a substituted or unsubstituted organic group optionally comprising one or more heteroatoms,
each of R₄ to R₁₁ is independently hydrogen atom, or a substituted or unsubstituted, saturated or unsaturated hydrocarbon group,
Me is methyl group, and
each of a, b, c and d is independently an integer of from 1 to 20.

As used herein, the expression "substituted or unsubstituted" for any group means that, unless specified otherwise, the group is not substituted, or is substituted with hydroxy group or C₁₋₆ alkyl group.

According to an embodiment of the present invention, in the above formula (1), each of M₁ and M₂ may be independently selected from the group consisting of -C(O)O-, -OC(O)-, - OC(O)-M'-C(O)O-, -C(O)N(R')-, -N(R')C(O)-, -C(O)-, -C(S)-, -C(S)S-, -SC(S)-, -CH(OH)-, - P(O)(OR')O-, -S(O)₂-, -S-S-, arylene (more concretely C₆₋₂₀ arylene, still more concretely C₆₋₁₀ arylene), and heteroarylene (more concretely C₃₋₂₀ heteroarylene, still more concretely C₃₋₁₀ heteroarylene, having one or more (e.g., 1 to 3) heteroatoms selected from N, O and S), wherein M' may be a direct bond, C₁₋₁₃ alkylene (more concretely C₁₋₆ alkylene) or C₂₋₁₃ alkenylene (more concretely C₂₋₆ alkenylene), and each R' may be independently selected from the group consisting of hydrogen atom, C₁₋₁₈ alkyl (more concretely C₁₋₁₀ alkyl, still more concretely C₁₋₆ alkyl) and C₂₋₁₈ alkenyl (more concretely C₂₋₁₀ alkenyl, still more concretely C₂₋₆ alkenyl).

According to an embodiment of the present invention, in the above formula (1), each of R₁ and R₂ may be independently selected from the group consisting of substituted or unsubstituted C₃₋₂₀ cycloalkyl (more concretely C₃₋₁₀ cycloalkyl, still more concretely C₃₋₆ cycloalkyl), substituted or unsubstituted C₃₋₂₀ cycloalkenyl (more concretely C₃₋₁₀ cycloalkenyl, still more concretely C₃₋₆ cycloalkenyl), substituted or unsubstituted C₆₋₂₀ aryl (more concretely C₆₋₁₀ aryl, still more concretely C₆ aryl), substituted or unsubstituted C₃₋₂₀ heterocycloalkyl (more concretely C₃₋₁₀ heterocycloalkyl, still more concretely C₃₋₆ heterocycloalkyl), substituted or unsubstituted C₃₋₂₀ heterocycloalkenyl (more concretely C₃₋₁₀ heterocycloalkenyl, still more concretely C₃₋₆ heterocycloalkenyl), and substituted or unsubstituted C₃₋₂₀ heteroaryl (more concretely C₃₋₁₀ heteroaryl, still more concretely C₃₋₆ heteroaryl), wherein each of the heterocycloalkyl, heterocycloalkenyl and heteroaryl may independently have one or more (e.g., 1 to 3) heteroatoms selected from N, O and S.

According to an embodiment of the present invention, in the above formula (1), R₃ may be selected from the group consisting of hydrogen atom, substituted or unsubstituted C₁₋₆ alkyl, substituted or unsubstituted C₃₋₆ carbocyclic group, -(CH₂)_{nQ}, - (CH₂)ₙCHQR, -CHQR and - CQ(R)₂, wherein each R may be independently selected from the group consisting of hydrogen atom, C₁₋₃ alkyl and C₂₋₃ alkenyl; Q may be selected from the group consisting of carbocyclic group, heterocyclic group, -OR, -O(CH₂)ₙN(R)₂, -C(O)OR, -OC(O)R, -CX₃, -CX₂H, -CXH₂, -CN, -N(R)₂, -C(O)N(R)₂, -N(R)C(O)R, -N(R)S(O)₂R, -N(R)C(O)N(R)₂, -N(R)C(S)N(R)₂, -N(R)R₁₂, N(R)S(O)2R₁₂, -O(CH2)ₙOR, -N(R)C(=NR₁₃)N(R)₂, -N(R)C(=CHR₁₃)N(R)₂, -OC(O)N(R)₂, - N(R)C(O)OR, -N(OR)C(O)R, -N(OR)S(O)₂R, -N(OR)C(O)OR, -N(OR)C(O)N(R)₂, - N(OR)C(S)N(R)₂, -N(OR)C(=NR₁₃)N(R)₂, -N(OR)C(=CHR₁₃)N(R)₂, -C(=NR₁₃)N(R)₂, - C(=NR₁₃)R, -C(O)N(R)OR and -C(R)N(R)₂C(O)OR, wherein each n is independently an integer of from 1 to 5; R₁₂ is selected from the group consisting of C₃₋₆ carbocyclic group and heterocyclic group; R₁₃ is selected from the group consisting of H, CN, NO₂, C₁₋₆ alkyl, -OR, -S(O)₂R, - S(O)₂N(R)₂, C₂₋₆ alkenyl, C₃₋₆ carbocyclic group and heterocyclic group; each R is independently selected from the group consisting of hydrogen atom, C₁₋₃ alkyl and C₂₋₃ alkenyl; each X is independently selected from the group consisting of F, CI, Br and I, provided that when R₃ is - (CH₂)ₙQ, -(CH₂)ₙCHQR, -CHQR or -CQ(R)₂, (i) if n is 1, 2, 3, 4, or 5, then Q is not -N(R)₂, or (ii) if n is 1 or 2, Q is not 5-, 6- or 7-membered heterocycloalkyl.

According to an embodiment of the present invention, in the above formula (1), each of R₄ to R₁₁ may be independently selected from the group consisting of hydrogen atom, C₁₋₃ alkyl and C₂₋₃ alkenyl.

According to an embodiment of the present invention, in the above formula (1), each of a, b, c and d may be independently an integer of from 1 to 15.

Still more concretely, in the above formula (1), each of M₁ and M₂ may be independently -C(O)O- or -OC(O)-.

Still more concretely, in the above formula (1), each of R₁ and R₂ may be independently substituted or unsubstituted C₃₋₆ cycloalkyl.

Still more concretely, in the above formula (1), R₃ may be hydrogen atom, or substituted or unsubstituted C₁₋₃ alkyl, and even more concretely, unsubstituted C₁₋₃ alkyl or hydroxy-substituted C₁₋₃ alkyl.

Still more concretely, in the above formula (1), R₄ to R₁₁ may be hydrogen atom.

Still more concretely, in the above formula (1), each of a, b, c and d may be independently an integer of from 3 to 11, and even more concretely, an integer of from 5 to 9.

Even more concretely, the lipid may be one having a structure selected from the following formulas A to O:

| Formula | Structure |
|---|---|
| A | 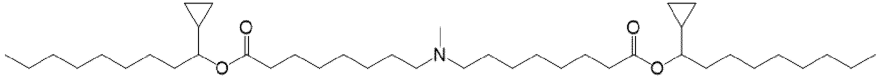 |
| B | 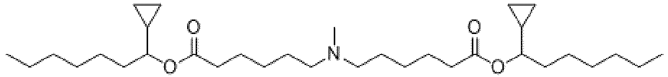 |
| C | 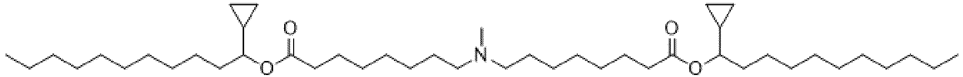 |
| D | 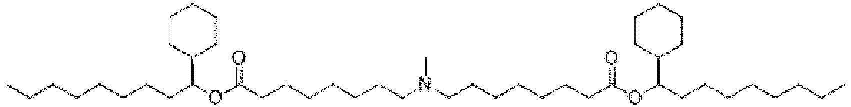 |
| E | 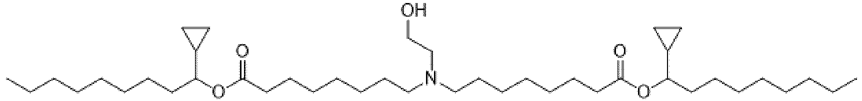 |
| F | 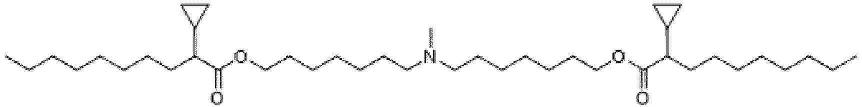 |
| G | 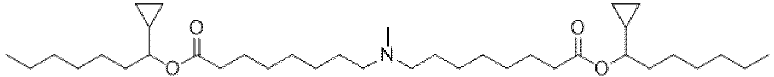 |
| H | 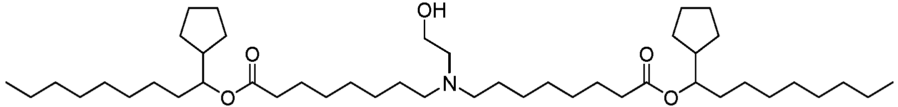 |
| I | 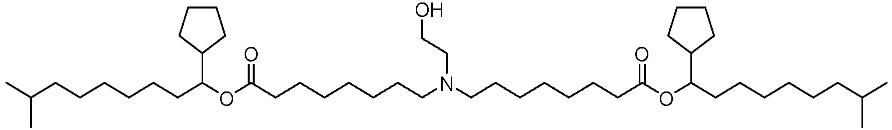 |
| J | 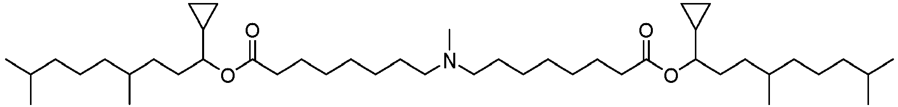 |
| K | 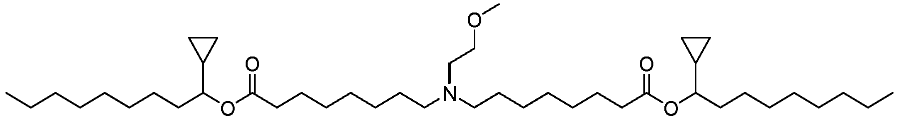 |
| L | 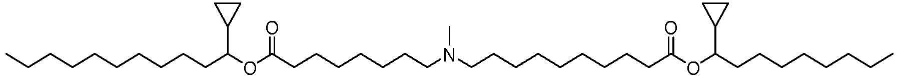 |
| M | 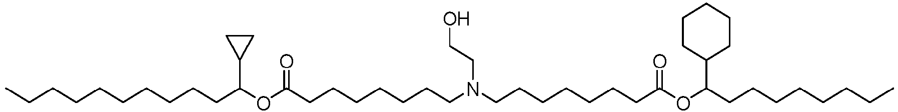 |
| N | 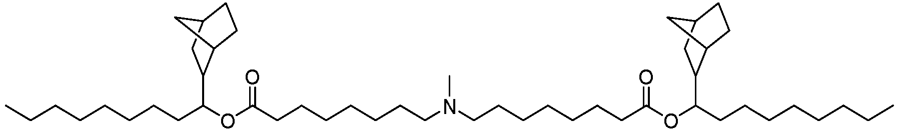 |
| O | 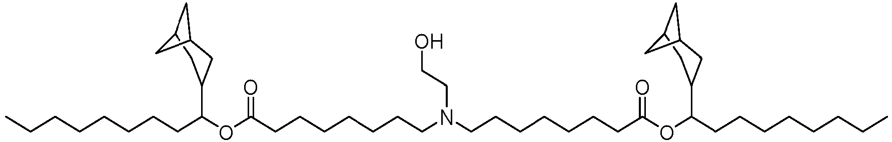 |

The second aspect of the present invention provides a method for preparing a lipid having a structure represented by formula (1') which is included in the above formula (1), comprising steps of: (1) reacting a compound of formula (a) with a compound of formula (b) to obtain a compound of formula (c); (2) reacting a compound of formula (c) with a compound of formula (d) to obtain a compound of formula (e); and (3) reacting a compound of formula (e) with a compound of formula (f):

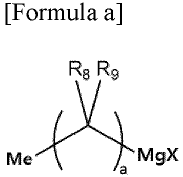

[Formula b] R₁-CHO

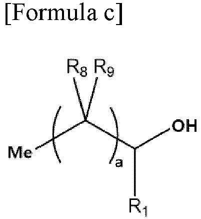

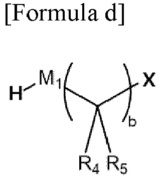

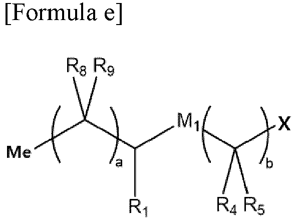

[Formula f] R₃-NH2

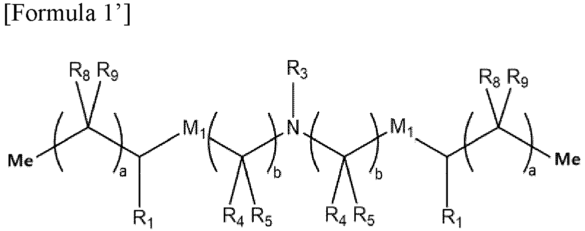

wherein,
M₁, R₁, R₃, R₄, R₅, R₈, R₉, Me, a and b are the same as defined in formula (1) above, and
each X is independently selected from the group consisting of F, CI, Br and I.

In an embodiment of the method for preparing a lipid according to the second aspect of the present invention, the reaction of step (1) may be conducted in a solvent (e.g., tetrahydrofuran (THF)) at a low temperature to room temperature (e.g., from -75°C to 30°C) condition; the reaction of step (2) may be conducted in a solvent (e.g., methylene chloride (DCM)) in the presence of a catalyst (e.g., 1-ethyl-3-(3-dimethylaminopropyl)carbodiimide (EDCI), 4-dimethylaminopyridine (DMAP), triethylamine (Et3N, TEA) or a combination thereof) at room temperature (e.g., from 20°C to 30°C) condition; and the reaction of step (3) may be conducted in a solvent (e.g., ethanol (EtOH) or THF) optionally in the presence of Na₂CO₃ or a catalyst (e.g., N,N-diisopropylethylamine (DIEA)) at an elevated temperature (e.g., from 40°C to 100°C) condition, but it is not limited thereto.

The third aspect of the present invention provides a method for preparing a lipid having a structure represented by the above formula (1), comprising steps of: (1) reacting a compound of formula (a') with a compound of formula (b') to obtain a compound of formula (c'); (2) reacting a compound of formula (c') with a compound of formula (d') to obtain a compound of formula (e'); and (3) reacting a compound of formula (e') and a compound of formula (e) obtained from the second aspect of the present invention with a compound of formula (f):

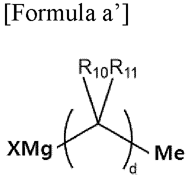

[Formula b'] R₂-CHO

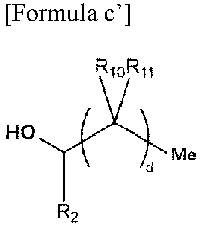

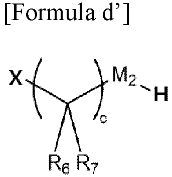

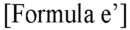

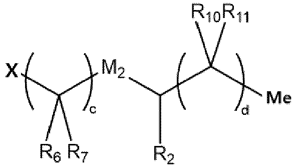

[Formula f] R₃-NH₂

wherein,
M₂, R₂, R₃, R₆, R₇, R₁₀, R₁₁, Me, c and d are the same as defined in formula (1) above, and
each X is independently selected from the group consisting of F, CI, Br and I.

In an embodiment of the method for preparing a lipid according to the third aspect of the present invention, the reaction of step (1) may be conducted in a solvent (e.g., tetrahydrofuran (THF)) at a low temperature to room temperature (e.g., from -75°C to 30°C) condition; the reaction of step (2) may be conducted in a solvent (e.g., methylene chloride (DCM)) in the presence of a catalyst (e.g., 1-ethyl-3-(3-dimethylaminopropyl)carbodiimide (EDCI), 4-dimethylaminopyridine (DMAP), triethylamine (Et3N, TEA) or a combination thereof) at room temperature (e.g., from 20°C to 30°C) condition; and the reaction of step (3) may be conducted in a solvent (e.g., ethanol (EtOH) or THF) optionally in the presence of Na₂CO₃ or a catalyst (e.g., N,N-diisopropylethylamine (DIEA)) at an elevated temperature (e.g., from 40°C to 100°C) condition, but it is not limited thereto.

The fourth aspect of the present invention provides a method for preparing a lipid having a structure represented by the above formula (1'), comprising steps of: (1) reacting a compound of formula (i) with a compound of formula (ii) to obtain a compound of formula (iii); (2) reacting a compound of formula (iii) with a compound of formula (iv) to obtain a compound of formula (v); (3) reacting a compound of formula (v) with a compound of formula (vi) to obtain a compound of formula (vii); and (4) reacting a compound of formula (v) with a compound of formula (vii):

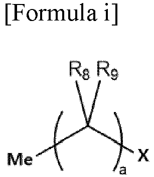

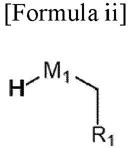

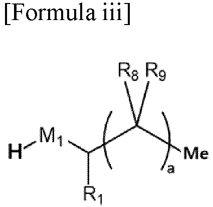

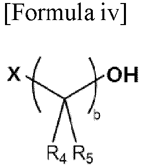

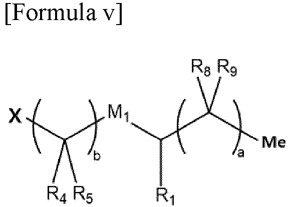

[Formula vi] R₃-NH;

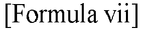

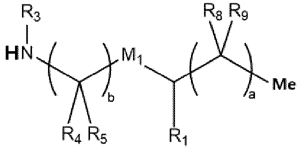

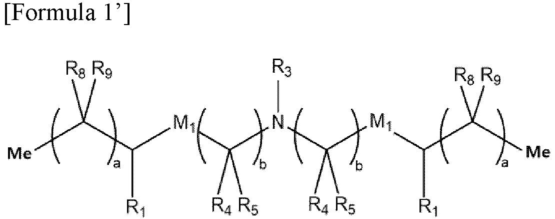

wherein,
M₁, R₁, R₃, R₄, R₅, R₈, R₉, Me, a and b are the same as defined in formula (1) above, and
each X is independently selected from the group consisting of F, CI, Br and I.

In an embodiment of the method for preparing a lipid according to the fourth aspect of the present invention, the reaction of step (1) may be conducted in a solvent (e.g., tetrahydrofuran (THF)) in the presence of a catalyst (e.g., sodium hydride (NaH) or lithium diisopropylamide (LDA)) at a low temperature to an elevated temperature (e.g., from -20°C to 60°C) condition; the reaction of step (2) may be conducted in a solvent (e.g., methylene chloride (DCM)) in the presence of a catalyst (e.g., 1-ethyl-3-(3-dimethylaminopropyl)carbodiimide (EDCI), 4-dimethylaminopyridine (DMAP), or a combination thereof) at room temperature (e.g., from 20°C to 30°C) condition; the reaction of step (3) may be conducted at an elevated temperature (e.g., from 40°C to 70°C) condition; and the reaction of step (4) may be conducted in a solvent (e.g., dioxane) optionally in the presence of Na₂CO₃ at an elevated temperature (e.g., from 40°C to 120°C) condition, but it is not limited thereto.

The fifth aspect of the present invention provides a method for preparing a lipid having a structure represented by formula (1), comprising steps of: (1) reacting a compound of formula (i') with a compound of formula (ii') to obtain a compound of formula (iii'); (2) reacting a compound of formula (iii') with a compound of formula (iv') to obtain a compound of formula (v'); (3) reacting a compound of formula (v') with a compound of formula (vi) to obtain a compound of formula (vii'); and (4) reacting a compound of formula (vii') with a compound of formula (v) obtained from the fourth aspect of the present invention: 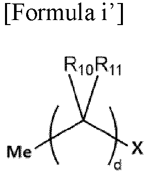 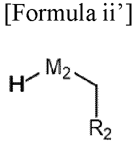 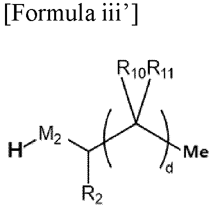 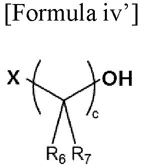 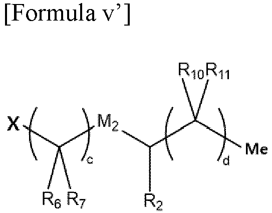 [Formula vi] R₃-NH2

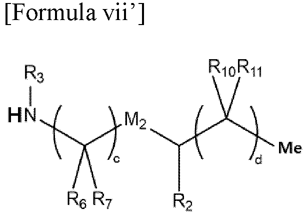

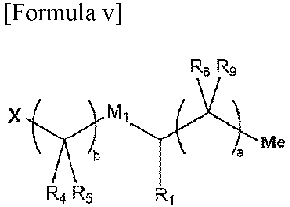

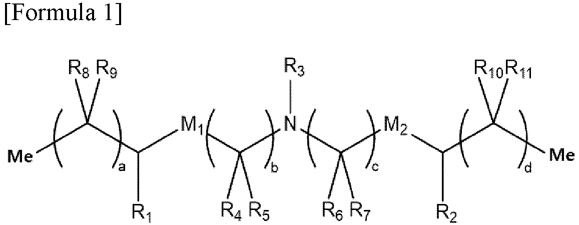

wherein,
M₁, M₂, R₁, R₂, R₃, R₄ to R₁₁, Me, a, b, c and d are the same as defined in formula (1) above, and
each X is independently selected from the group consisting of F, CI, Br and I.

In an embodiment of the method for preparing a lipid according to the fifth aspect of the present invention, the reaction of step (1) may be conducted in a solvent (e.g., tetrahydrofuran (THF)) in the presence of a catalyst (e.g., sodium hydride (NaH) or lithium diisopropylamide (LDA)) at a low temperature to an elevated temperature (e.g., from -20°C to 60°C) condition; the reaction of step (2) may be conducted in a solvent (e.g., methylene chloride (DCM)) in the presence of a catalyst (e.g., 1-ethyl-3-(3-dimethylaminopropyl)carbodiimide (EDCI), 4-dimethylaminopyridine (DMAP), or a combination thereof) at room temperature (e.g., from 20°C to 30°C) condition; the reaction of step (3) may be conducted at an elevated temperature (e.g., from 40°C to 70°C) condition; and the reaction of step (4) may be conducted in a solvent (e.g., dioxane) optionally in the presence of Na₂CO₃ at an elevated temperature (e.g., from 40°C to 120°C) condition, but it is not limited thereto.

A lipid having a structure represented by formula 1 of the present invention can easily form a complex with anionic drug and thus is useful for drug delivery.

Thus, the sixth aspect of the present invention provides a composition for drug delivery comprising a lipid having a structure represented by formula 1 of the present invention.

In an embodiment, the drug may be selected from nucleic acid, polypeptide, virus or combination thereof.

The "nucleic acid" may be, for example, DNA, RNA, siRNA, shRNA, miRNA, mRNA, aptamer, antisense oligonucleotide, or a combination thereof, but it is not limited thereto.

The "polypeptide" may mean a protein having activity in the body such as antibody or fragment thereof, cytokine, hormone or analog thereof, or a protein that can be recognized as antigen through a series of processes in the body, including polypeptide sequence of antigen, analog or precursor thereof.

In an embodiment, the lipid of the present invention may form a complex with the drug, and the complex is encapsulated within nanoparticle structure formed by an amphiphilic block copolymer.

In an embodiment, the amphiphilic block copolymer may be an A-B type block copolymer comprising a hydrophilic A block and a hydrophobic B block. In an aqueous environment, the A-B type block copolymer forms core-shell type polymer nanoparticle wherein the hydrophobic B block forms the core (inner wall) and the hydrophilic A block forms the shell (outer wall).

In an embodiment, the hydrophilic A block may be one or more selected from the group consisting of polyalkylene glycol, polyvinyl alcohol, polyvinyl pyrrolidone, polyacrylamide, and derivatives thereof.

More concretely, the hydrophilic A block may be one or more selected from the group consisting of monomethoxypolyethylene glycol (mPEG), monoacetoxypolyethylene glycol, polyethylene glycol, copolymer of polyethylene and propylene glycol, and polyvinylpyrrolidone.

Also, if necessary, the end of the hydrophilic A block may be chemically combined with a functional group or ligand capable of reaching specific tissue or cell, or a functional group capable of promoting intracellular delivery, in order to control *in vivo* distribution of polymer nanoparticle carrier formed by the amphiphilic block copolymer and salt of polylactic acid or to increase the efficiency of delivering the nanoparticle carrier into cell. In an embodiment, the functional group or ligand may be one or more selected from the group consisting of monosaccharides, polysaccharides, vitamins, peptides, proteins, and antibodies to cell surface receptors. More concretely, the functional group or ligand may be one or more selected from the group consisting of anisamide, vitamin B9 (folic acid), vitamin B12, vitamin A, galactose, lactose, mannose, hyaluronic acid, RGD peptide, NGR peptide, transferrin, antibody to transferrin receptor, etc.

The hydrophobic B block is a biocompatible, biodegradable polymer, and in an embodiment, it may be one or more selected from the group consisting of polyester, polyanhydride, polyamino acid, polyorthoester and polyphosphazine.

More concretely, the hydrophobic B block may be one or more selected from the group consisting of polylactide (PLA), polyglycolide, polycaprolactone, polydioxan-2-one, copolymer of polylactide and glycolide, copolymer of polylactide and polydioxan-2-one, copolymer of polylactide and and polycaprolactone, and a copolymer of polyglycolide and polycaprolactone.

Also, in an embodiment, in order to increase the hydrophobicity of the hydrophobic B block and thereby improve the stability of the nanoparticle, the hydrophobic B block may be modified by chemically combining the hydroxyl group at the end of the hydrophobic B block with tocopherol, cholesterol, or fatty acid having 10 to 24 carbons.

The present invention will be explained below in more detail with reference to the following Examples. However, the Examples are only to illustrate the invention, and the scope of the present invention is not limited thereby in any manner.

### EXAMPLES

### Example 1

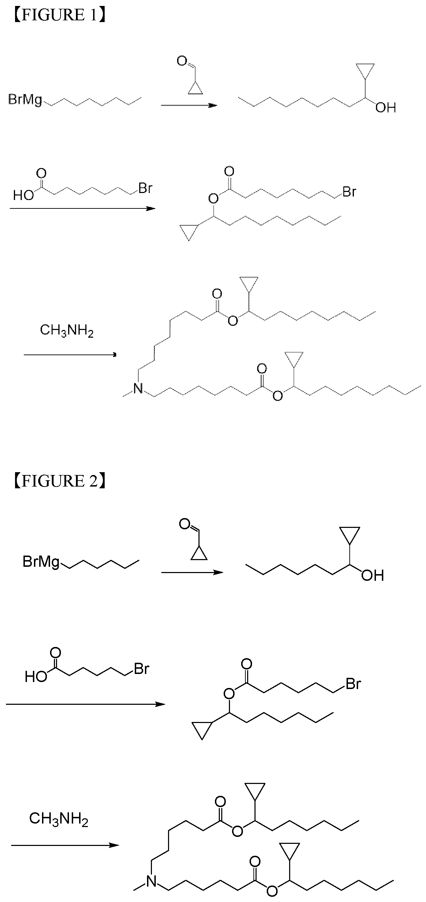

The compound of the following formula A was prepared according to the synthesis scheme shown in Figure 1.

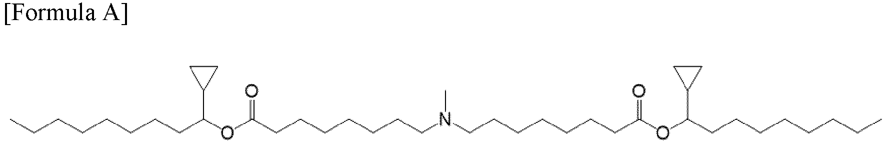

### (1) Synthesis of 1-cyclopropylnonan-1-ol

In a 2000 mL 3-neck round bottom flask (RBF), cyclopropanecarbaldehyde (35.0 g, 499 mmol, 1.00 eq) and tetrahydrofuran (THF) (700 mL) were added under a nitrogen environment and cooled to -65°C, and then octylmagnesium bromide (2 M, 375 mL, 1.50 eq) was added, and the mixture was stirred at -65°C for 2 hours. The reactor was heated to 15°C, then the reaction mixture was poured into a saturated NH₄Cl aqueous solution (500 mL), and the organic layer and aqueous layer were separated. The aqueous layer was extracted with ethyl acetate (EtOAc) (450 mL) (150 mL each, three times). The organic layers were collected, concentrated in vacuo, and purified using a silica column with petroleum ether:EtOAc = 50:1 → 0:1 to obtain 1-cyclopropylnonan-1-ol (87.5 g, 73.1%).
¹H NMR (400 MHz, CHLOROFORM-*d*): *δ* 2.93 - 2.81 (m, 1H), 1.61 (br d, 2H), 1.52 - 1.27 (m, 12H), 0.95 - 0.86 (m, 4H), 0.60 - 0.45 (m, 2H), 0.34 - 0.19 (m, 2H)

### (2) Synthesis of 1-cyclopropylnonyl 8-bromooctanoate

In a 1000 mL 3-neck RBF, 1-cyclopropylnonan-1-ol (30.0 g, 163 mmol, 1.00 eq), 8-bromooctanoic acid (72.6 g, 326 mmol, 2.00 eq), methylene chloride (DCM) (300 mL), 1-ethyl-3-(3-dimethylaminopropyl)carbodiimide (EDCI) (31.2 g, 163 mmol, 1.00 eq), and 4-dimethylaminopyridine (DMAP) (19.9 g, 163 mmol, 1.00 eq) were added, and the mixture was stirred at 25°C for 16 hours. The reaction mixture was concentrated in vacuo, and after adding silica powder thereto, it was purified using a silica column with petroleum ether:EtOAc = 10:1 → 50:1 to obtain 1-cyclopropylnonyl 8-bromooctanoate (22.8 g, 36.0%).
¹H NMR (400 MHz, CHLOROFORM-*d*): *δ* 4.29 (td, 1H), 3.59 - 3.31 (m, 2H), 2.32 (t, 2H), 1.94 - 1.75 (m, 2H), 1.73 - 1.60 (m, 4H), 1.49 - 1.25 (m, 18H), 1.03 - 0.93 (m, 1H), 0.90 (t, 3H), 0.61 - 0.24 (m, 4H)

### (3) Synthesis of the compound of formula A

In a 100 mL 3-neck flask, methylamine hydrochloride (173 mg, 2.57 mmol, 1.00 eq), ethanol (EtOH) (30 mL), N,N-diisopropylethylamine (DIEA) (1.66 g, 12.8 mmol, 5.00 eq), and 1-cyclopropylnonyl 8-bromooctanoate (3.00 g, 7.70 mmol, 3.00 eq) were added sequentially, and the mixture was stirred at 80°C for 72 hours. The reaction mixture was concentrated in vacuo, and after adding silica powder thereto, it was purified using a silica column with petroleum ether:EtOAc = 10:1 → 1:1 to obtain the compound of formula A (660 mg, 38.9%).
¹H NMR (400 MHz, CHLOROFORM-*d*): *δ* 4.29 (td, 2H), 2.32 (br t, 8H), 2.22 (s, 3H), 1.74 - 1.60 (m, 8H), 1.54 - 1.42 (m, 4H), 1.39 - 1.23 (m, 36H), 1.02 - 0.87 (m, 8H), 0.61 - 0.23 (m, 8H)

### Example 2

The compound of the following formula B was prepared according to the synthesis scheme shown in Figure 2.

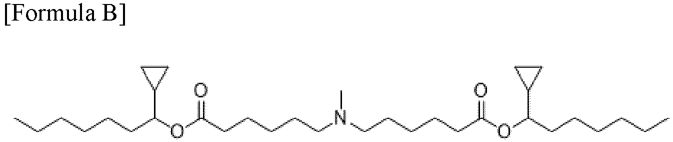

### (1) Synthesis of 1-cyclopropylheptan-1-ol

In a 2000 mL 3-neck RBF, cyclopropanecarbaldehyde (27.0 g, 385 mmol, 1.00 eq) and THF (500 mL) were added under a nitrogen environment, cooled to -65°C, and hexylmagnesium bromide (1 M, 500 mL, 1.30 eq) was slowly added. The mixture was stirred at -65°C for 3 hours, and then the temperature of the reactor was slowly increased to 25°C. Then, the mixture was poured into a saturated NH₄Cl aqueous solution (500 mL), and the organic layer and aqueous layer were separated. The aqueous layer was extracted with EtOAc (500 mL each, three times). The organic layers were collected and concentrated in vacuo, and the residue after concentration was purified using a silica column with petroleum ether:EtOAc = 50:1 → 10:1 to obtain 1-cyclopropylheptan-1-ol (46.0 g, 294 mmol, 76.4% yield) as a colorless oil.
¹H NMR (400 MHz, CHLOROFORM-d): δ 2.86 (td, 1H), 1.67 - 1.38 (m, 6H), 1.36 - 1.27 (m, 6H), 0.91 - 0.87 (m, 3H), 0.59 - 0.42 (m, 2H), 0.32 - 0.17 (m, 2H)

### (2) Synthesis of 1-cyclopropylheptyl 6-bromohexanoate

In a 500 mL 3-neck RBF, 1-cyclopropylheptan-1-ol (10.0 g, 63.9 mmol, 1.00 eq), 6-bromohexanoic acid (12.5 g, 63.9 mmol, 1.00 eq), DCM (100 mL), EDCI (15.9 g, 83.2 mmol, 1.30 eq), and DMAP (10.2 g, 83.2 mmol, 1.30 eq) were added, and the mixture was stirred at 25 °C for 16 hours. The reaction mixture was concentrated in vacuo, and purified using a silica column with petroleum ether:EtOAc = 50:1 → 10:1 to obtain 1-cyclopropylheptyl 6-bromohexanoate (7.50 g, 22.5 mmol, 35.1%)
¹H NMR: (400 MHz, CHLOROFORM-d): δ 4.28 (td, 1H), 3.55 (t, 1H), 3.42 (t, 1H), 2.39 - 2.26 (m, 2H), 1.97 - 1.76 (m, 2H), 1.71 - 1.60 (m, 4H), 1.57 - 1.42 (m, 3H), 1.33 - 1.28 (m, 6H), 0.91 - 0.86 (m, 5H), 0.63 - 0.51 (m, 1H), 0.50 - 0.41 (m, 1H), 0.40 - 0.32 (m, 1H), 0.30 - 0.22 (m, 1H)

### (3) Synthesis of the compound of formula B

In a 100 mL 3-neck RBF, 1-cyclopropylheptyl 6-bromohexanoate (500 mg, 1.00 eq) was placed, and methylamine in THF (2 M, 2 g, 42.8 eq) was added under nitrogen environment, and then the mixture was stirred at 50°C for 16 hours. To the stirred reaction mixture, a sodium carbonate (Na₂CO₃) aqueous solution (20 mL) was added and stirred for 2 hours to adjust the pH to 8. Then, extraction was performed with DCM (30 mL*3) and the organic layer was preserved. The preserved organic layer was concentrated in vacuo and purified using a silica column with DCM: methanol = 10:1 to obtain the compound of formula B (68 mg, 8.56%) as a yellow oil.
¹H NMR (400 MHz, CHLOROFORM-d) *δ* 4.27 (td, 2H), 2.43 - 2.16 (m, 10H), 1.75 - 1.62 (m, 8H), 1.55 - 1.43 (m, 5H), 1.40 - 1.20 (m, 22H), 1.00 - 0.91 (m, 2H), 0.90 - 0.85 (m, 4H), 0.62 - 0.50 (m, 2H), 0.49 - 0.33 (m, 4H), 0.30 - 0.21 (m, 2H)

### Example 3

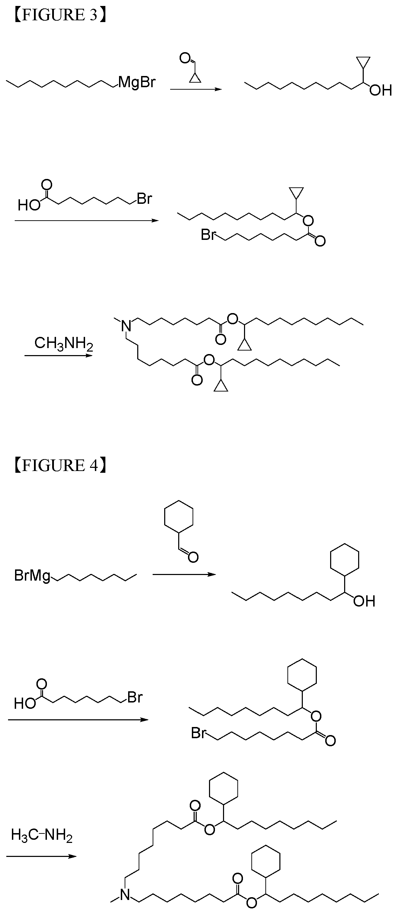

The compound of the following formula C was prepared according to the synthesis scheme shown in Figure 3.

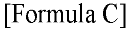

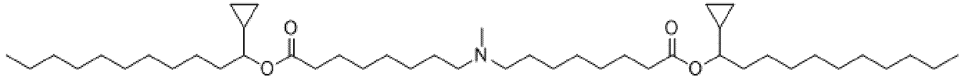

### (1) Synthesis of 1-cyclopropylundecan-1-ol

In a 2000 mL 3-neck RBF, cyclopropanecarbaldehyde (27.0 g, 385 mmol, 1.00 eq) and THF (270 mL) were added, and the mixture was purged with nitrogen three times, cooled to -60°C, and then decylmagnesium bromide (1 M, 501 mL, 1.30 eq) was added, and the mixture was stirred at -60 °C for 16 hours in a nitrogen environment. The reactor was heated to 25°C, and the mixture was poured into a saturated NH₄Cl aqueous solution (200 mL) to separate the organic and aqueous layers. The aqueous layer was extracted with EtOAc (100 mL each, 4 times), and the organic layer was collected, concentrated in vacuo and purified using a silica column with petroleum ether:EtOAc = 10:1 to obtain 1-cyclopropylundecan-1-ol (56.0 g, 68.5%).
¹H NMR (400 MHz, CHLOROFORM-d): *δ* 0.17 - 0.31 (m, 2 H) 0.43 - 0.57 (m, 2 H) 0.88 (t, 4H) 1.26 (br s, 14 H) 1.37 - 1.49 (m, 2 H) 1.54 - 1.64 (m, 3 H) 2.85 (dt, 1 H)

### (2) Synthesis of 1-cyclopropylundecyl 8-bromooctanoate

In a 1000 mL 3-neck RBF, 1-cyclopropylundecan-1-ol (24.0 g, 113 mmol, 1.00 eq), 8-bromooctanoic acid (32.8 g, 147 mmol, 1.30 eq), DCM (300 mL), EDCI (26.0 g, 136 mmol, 1.20 eq), and DMAP (16.6 g, 136 mmol, 1.20 eq) were added, and the mixture was purged with nitrogen three times. The mixture was stirred at 25°C for 16 hours and then poured into water (200 mL), and the organic layer and the aqueous layer were separated. The aqueous layer was extracted with DCM (200 mL each, three times). The extracted organic layer was dried over Na₂SO₄, and the dried mixture was filtered and concentrated in vacuo. The residue after concentration was purified using a silica column with petroleum ether:EtOAc=10:1→1:1 to obtain 1-cyclopropylundecyl 8-bromooctanoate (11.0 g, 23.3%).
¹H NMR (400 MHz, CHLOROFORM-d): *δ* 0.22 - 0.30 (m, 1 H) 0.33 - 0.40 (m, 1 H) 0.42 - 0.49 (m, 1 H) 0.50 - 0.58 (m, 1 H) 0.85 - 1.00 (m, 4 H) 1.20 - 1.38 (m, 20 H) 1.41 - 1.49 (m, 2 H) 1.58 - 1.68 (m, 4 H) 1.73 - 1.91 (m, 2 H) 2.25 - 2.37 (m, 2 H) 3.35 - 3.56 (m, 2 H) 4.21 - 4.35 (m, 1 H)

### (3) Synthesis of the compound of formula C

In a 100 mL 3-neck RBF, 1-cyclopropylundecyl 8-bromooctanoate (3.00 g, 7.19 mmol, 1.00 eq) and methylamine solution (2 M in THF, 10.3 g, 331 mmol, 46.0 eq) were, and then the mixture was stirred at 50°C for 16 hours. After concentrating the mixture in the reactor in vacuo, the pH was adjusted by adding NaHCO₃ aqueous solution. Then, the mixture was extracted with DCM, and the extracted organic layer was concentrated in vacuo. The residue after concentration was purified using a silica column with DCM:methanol = 10:1 → 1:1 to obtain the compound of formula C (2.00 g, 63.0%) as a yellow oil.
¹H NMR (400 MHz, CHLOROFORM-*d*) *δ* 0.21 - 0.30 (m, 2 H) 0.37 (dq, 2 H) 0.42 - 0.49 (m, 2 H) 0.50 - 0.58 (m, 2 H) 0.84 - 0.98 (m, 8 H) 1.24 - 1.36 (m, 44 H) 1.51 (br s, 4 H) 1.59 - 1.68 (m, 8 H) 2.23 - 2.33 (m, 7 H) 2.34 - 2.44 (m, 4 H) 4.21 - 4.33 (m, 2 H)

### Example 4

The compound of the following formula D was prepared according to the synthesis scheme shown in Figure 4.

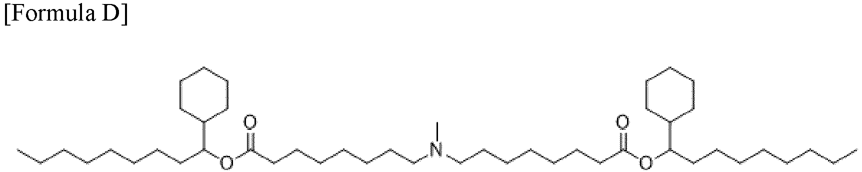

### (1) Synthesis of 1-cyclohexylnonan-1-ol

In a 1000 mL 3-neck RBF, cyclohexanecarbaldehyde (43.0 g, 383 mmol, 1.00 eq) and THF (430 mL) were added, and the mixture was purged three times with nitrogen, cooled to - 65 °C, and then octylmagnesium bromide (1 M in THF, 498 mL, 1.30 eq) was added, and the mixture was stirred at -65 °C for 1 hour in a nitrogen environment. The reactor was heated to 25°C, and the mixture was poured into a saturated NH₄Cl aqueous solution (700 mL) to separate the organic layer and the aqueous layer. The aqueous layer was extracted with EtOAc (400 mL each, three times), the organic layers were collected, concentrated in vacuo, and purified using a silica column with petroleum ether:EtOAc = 10:1→1:10 to obtain 1-cyclohexylnonan-1-ol (12.0 g, 53.0 mmol, 13.8%) as a colorless oil.
¹HNMR (400 MHz, CHLOROFORM-*d*): *δ* 3.41 - 3.31 (m, 1H), 1.84 - 1.73 (m, 3H), 1.70 - 1.63 (m, 2H), 1.48 (br d, 3H), 1.36 - 1.21 (m, 15H), 1.18 - 1.00 (m, 3H), 0.94 - 0.84 (m, 3H)

### (2) Synthesis of 1-cyclohexylnonyl 8-bromooctanoate

In a 250 mL 3-neck RBF, 1-cyclohexylnonan-1-ol (7.00 g, 30.9 mmol, 1.00 eq) was placed, and DCM (70 mL) was added. Then, 8-bromooctanoic acid (8.28 g, 37.1 mmol, 1.20 eq), EDCI (7.11 g, 37.1 mmol, 1.20 eq), DMAP (755 mg, 6.18 mmol, 0.20 eq), and Et3N (6.26 g, 61.8 mmol, 2.00 eq) were added and mixed. The mixture was stirred at 25°C for 16 hours, and purged with nitrogen three times. The reaction mixture was filtered using a celite plug, and the filtrate was concentrated in vacuo. The residue after concentration was purified using a silica column with petroleum ether:EtOAc = 10:1 → 1:100 to give 1-cyclohexylnonyl 8-bromooctanoate (3.50 g, 8.11 mmol, 26.2%) as a yellow oil.
¹HNMR (400 MHz, CHLOROFORM-*d*): *δ* 4.07 (t, 2H), 3.41 (t, 2H), 2.30 (t, 2H), 1.91 - 1.80 (m, 2H), 1.67 - 1.58 (m, 4H), 1.47 - 1.41 (m, 2H), 1.37 - 1.26 (m, 16H), 0.91 - 0.87 (m, 3H)

### (3) Synthesis of the compound of formula D

In a 100 mL 3-neck RBF, 1-cyclohexylnonyl 8-bromooctanoate (1.50 g, 3.48 mmol, 1.00 eq) was placed, and methylamine in THF (CH₃NH₂ in THF) (15.6 g, 151 mmol, 30% purity, 43.5 eq) was added. The mixture was purged with nitrogen three times and stirred at 50°C for 16 hours. After stirring, the reaction mixture was filtered, and the filtrate was concentrated in vacuo. The residue after concentration was purified using a silica column with DCM:MeOH = 100:1 → 10:1 to give the compound of formula D (0.13 g, 178 µmol, 5.11% yield) as a yellow oil.
¹H NMR (400 MHz, CHLOROFORM-*d*) *δ* 4.81 - 4.69 (m, 2H), 2.30 (br t, 8H), 2.22 (br s, 3H), 1.76 - 1.71 (m, 4H), 1.69 - 1.60 (m, 12H), 1.53 - 1.43 (m, 10H), 1.35 - 1.22 (m, 40H), 1.05 - 0.97 (m, 4H), 0.88 (br t, 6H)

### Example 5

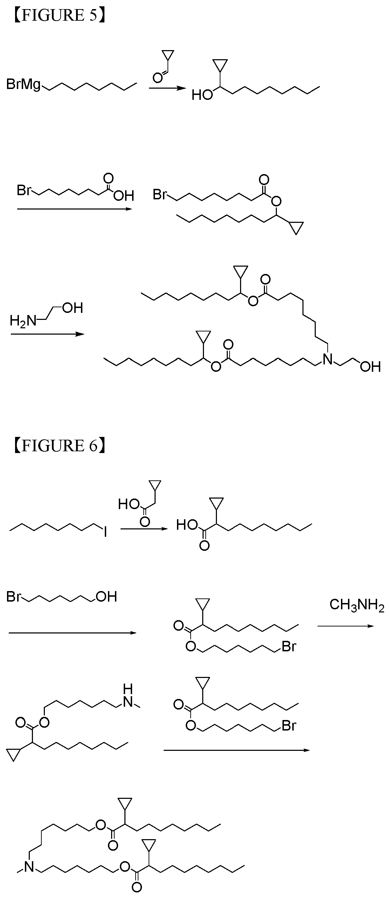

The compound of the following formula E was prepared according to the synthesis scheme shown in Figure 5.

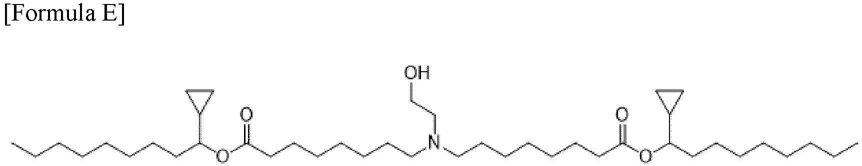

### (1) Synthesis of 1-cyclopropylnonan-1-ol

In a 1000 mL 3-neck RBF, cyclopropanecarbaldehyde (46.0 g, 656 mmol, 1.00 eq) was placed, THF was added, and then octylmagnesium bromide (2.00 M, 492 mL, 1.50 eq) was added under a nitrogen environment. The mixture was stirred at -65°C for 3 hours, and then saturated NH₄Cl aqueous solution (700 mL) was poured thereto at 15°C, and the organic layer and the aqueous layer were separated. The aqueous layer was additionally extracted with EtOAc (200 mL x 3). The organic layers were collected, concentrated in vacuo, and purified using a silica column with petroleum ether:EtOAc = 100:1 to obtain 1-cyclopropylnonan-1-ol (59.5 g, 49.2%) as a colorless oil.
¹H NMR (400 MHz, CHLOROFORM-*d*): *δ* 2.63 (td, 1H), 1.47 - 1.31 (m, 5H), 1.29 - 1.09 (m, 6H), 0.73 - 0.61 (m, 6H), 0.37 - 0.19 (m, 3H), 0.10 - 0.06 (m, 3H)

### (2) Synthesis of 1-cyclopropylnonyl 8-bromooctanoate

In a 1000 mL 3-neck RBF, DCM (600 mL), 1-cyclopropylnonan-1-ol (59.5 g, 325 mmol, 1.00 eq), 8-bromooctanoic acid (94.4 g, 423 mmol, 1.30 eq), EDCI (93.6 g, 488 mmol, 1.50 eq), DMAP (39.8 g, 326 mmol, 1.00 eq), and TEA (32.9 g, 326 mmol, 45.3 mL, 1.00 eq) were added, and the mixture was stirred at 25°C for 16 hours under a nitrogen environment. The reaction mixture was concentrated in vacuo to obtain a residue. The obtained residue was purified using a silica column with petroleum ether:EtOAc = 100:1 → 1:1. Through this process, 1-cyclopropylnonyl 8-bromooctanoate (21.0 g, 53.9 mmol, 16.6% yield) was obtained as a pale yellow oil.
¹H NMR (400 MHz, CHLOROFORM-d): *δ* 0.05 - 0.16 (m, 2 H) 0.16 - 0.34 (m, 2 H) 0.57 - 0.67 (m, 2 H) 0.67 -0.80 (m, 1 H) 1.02 (br s, 9 H) 1.05 (br s, 2 H) 1.07 - 1.15 (m, 6 H) 1.16 - 1.29 (m, 2 H) 1.34 - 1.44 (m, 4 H) 1.48 - 1.66(m, 2 H) 2.05 (t, 2 H) 3.06 - 3.33 (m, 2 H) 3.97 - 4.07 (m, 1 H)

### (3) Synthesis of the compound of formula E

In a 100 mL 3-neck RBF, 1-cyclopropylnonyl 8-bromooctanoate (4.78 g, 12.3 mmol, 2.50 eq), 2-aminoethanol (MEA) (0.30 g, 4.91 mmol, 1.00 eq), and Na₂CO₃ (521 mg, 4.91 mmol, 1.00 eq) were added in EtOH (5 mL), and the mixture was purged with nitrogen three times. The mixture was stirred at 95°C for 16 hours. The mixture in the reactor was concentrated in vacuo to obtain a residue. The residue was then purified using a silica column with DCM:MeOH = 100:1 → 10:1 to obtain the compound of formula E (1.00 g, 1.47 mmol, 30.0% yield) as a yellow oil.
¹H NMR (400 MHz, CHLOROFORM-*d*): *δ* = 4.27 (td, 2H), 3.53 (t, 2H), 2.57 (t, 2H), 2.47 - 2.40 (m, 4H), 2.30 (t, 4H), 1.70 - 1.57 (m, 10H), 1.48 - 1.39 (m, 4H), 1.36 - 1.26 (m, 33H), 0.95 (dt, 2H), 0.89 (t, 6H), 0.60 - 0.42 (m, 4H), 0.41 - 0.22 (m, 4H)

### Example 6

The compound of the following formula F was prepared according to the synthesis scheme shown in Figure 6.

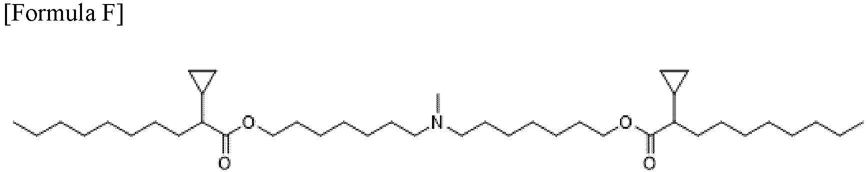

### (1) Synthesis of 2-cyclopropyldecanoic acid

In a 2000 mL 3-neck RBF, 2-cyclopropylacetic acid (25.0 g, 250 mmol, 1.00 eq) was added, followed by addition of THF (250 mL) and cooling with nitrogen. Then, sodium hydride (NaH) (11.0 g, 275 mmol, 60% purity, 1.10 eq) was added and the mixture was stirred at 0°C for 30 min. Then, lithium diisopropylamide (LDA) (2 M, 137 mL, 1.10 eq) was added at the same temperature and conditions, and the mixture was stirred for 30 min. Then, 1-iodooctane (60.0 g, 250 mmol, 1.00 eq) was added at 25°C, and the mixture was stirred at 45°C for 12 hours in a nitrogen environment. The reaction mixture was neutralized with 100 mL of water and 1 M HCl (600 mL, pH= 4), and then extracted with EtOAc (300 mL * 3). The extracted organic layer was dried over anhydrous Na₂SO₄ and concentrated in vacuo. The residue after concentration was purified using a silica column with petroleum ether:EtOAc = 20:1 → 5:1 to obtain 2-cyclopropyldecanoic acid (42.6 g, 201 mmol, 80.4% yield) as a yellow oil.
¹H NMR (400 MHz, CHLOROFORM-d): *δ* 11.88 (s, 1H), 1.84 - 1.70 (m, 1H), 1.70 - 1.54 (m, 2H), 1.42 - 1.19 (m, 12H), 1.00 - 0.81 (m, 4H), 0.63 - 0.45 (m, 2H), 0.32 (qd, 1H), 0.23 - 0.10 (m, 1H)

### (2) Synthesis of 7-bromoheptyl 2-cyclopropyldecanoate

In a 2000 mL 3-neck RBF, 2-cyclopropyldecanoic acid (10.0 g, 47.1 mmol, 1.00 eq), 7-bromoheptan-1-ol (11.0 g, 56.5 mmol, 1.20 eq), EDCI (11.7 g, 61.2 mmol, 1.30 eq), and DMAP (5.75 g, 47.1 mmol, 1.00 eq) were added together with DCM (100 mL), and the mixture was purged with nitrogen three times. The mixture was stirred at 25°C for 16 hours in a nitrogen environment, the reaction mixture was warmed to 25°C, and then poured into water (100 mL) to separate the organic layer and the aqueous layer. The aqueous layer was extracted with DCM (100 mL each, 3 times). The organic layers were collected, concentrated in vacuo, dried over anhydrous Na₂SO₄, and filtered. The filtered residue was purified using a silica column with petroleum ether:EtOAc = 20:1 → 5:1 to obtain 7-bromoheptyl 2-cyclopropyldecanoate (9.40 g, 24.1 mmol, 51.2%) as a pale yellow oil.
¹H NMR (400 MHz, CHLOROFORM-*d*): *δ* 4.10 (t, *J =* 6.6 Hz, 2H), 3.42 (t, 2H), 1.89 - 1.84 (m, 1H), 1.81 - 1.51 (m, 6H), 1.50 - 1.35 (m, 6H), 1.27 (br s, 12H), 0.89 (t, 4H), 0.61 - 0.37 (m, 2H), 0.24 (s, 1H), 0.13 (qd, 1H)

### (3) Synthesis of 7-(methylamino)heptyl 2-cyclopropyldecanoate

In a 100 mL 3-neck RBF, 7-bromoheptyl 2-cyclopropyldecanoate (4.00 g, 10.3 mmol, 1.00 eq) and methylamine (2 M in THF, 185 mL, 36.0 eq) were added, and the mixture was stirred at 50°C for 16 hours in a nitrogen atmosphere. The stirred reaction mixture was concentrated in vacuo, extracted with DCM (30 mL*3) and the organic layer was preserved. The preserved organic layer was concentrated in vacuo and purified using a silica column with DCM:methanol = 20:1 → 10:1 to obtain 7-(methylamino)heptyl 2-cyclopropyldecanoate (0.45 g, 1.33 mmol, 12.9%) as a yellow oil.
¹H NMR (400 MHz, CHLOROFORM-d): *δ* 4.19 - 3.97 (m, 2H), 2.59 (t, 2H), 2.45 (s, 3H), 1.79 - 1.69 (m, 1H), 1.67 - 1.46 (m, 6H), 1.43 - 1.19 (m, 18H), 0.88 (t, 4H), 0.60 - 0.40 (m, 2H), 0.30 - 0.06 (m, 2H)

### (4) Synthesis of the compound of formula F

In a 50 mL 3-neck RBF, 7-(methylamino)heptyl 2-cyclopropyldecanoate (0.40 g, 1.18 mmol, 1.00 eq) was placed, and 7-bromoheptyl 2-cyclopropyldecanoate (0.60 g, 1.53 mmol, 1.30 eq) and Na₂CO₃ (0.25 g, 2.36 mmol, 2.00 eq) in dioxane (2 mL) were added. The mixture was stirred at 100°C for 16 hours, and then cooled and poured into water (5 mL) to separate the organic layer and the aqueous layer. The aqueous layer was extracted with EtOAc (5 mL x 3), and the organic layers were collected and concentrated in vacuo, and then washed with saturated Na₂CO₃ aqueous solution (5 mL), dried over anhydrous Na₂SO₄, and filtered. After concentration, the residue was purified using a silica column with DCM:methanol = 20:1 → 10:1 to obtain the compound of formula F (0.36 g, 556 µmol, 47.2%) as a pale yellow oil.
¹H NMR: (400 MHz, CHLOROFORM-*d*) *δ* 4.09 (dt, 4H), 2.72 - 2.07 (m, 7H), 1.73 (br dd, 2H), 1.67 - 1.50 (m, 12H), 1.40 - 1.23 (m, 36H), 0.88 (br t, 8H), 0.59 - 0.50 (m, 2H), 0.45 (s, 2H), 0.24 (s, 2H), 0.13 (br d, 2H)

### Example 7

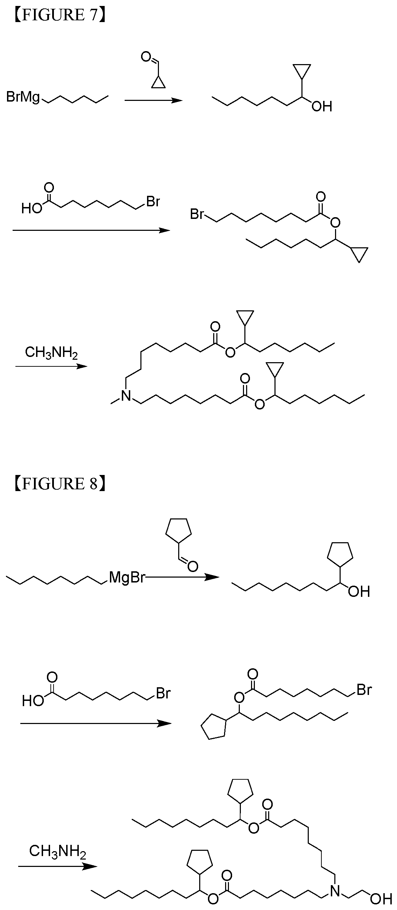

The compound of the following formula G was prepared according to the synthesis scheme shown in Figure 7.

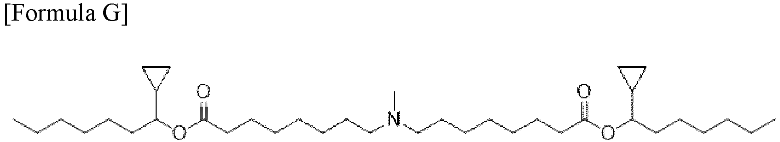

### (1) Synthesis of 1-cyclopropylheptan-1-ol

In a 2000 mL 3-neck RBF, cyclopropanecarbaldehyde (27.0 g, 385 mmol, 1.00 eq) was placed together with THF (500 mL). To this mixture, hexylmagnesium bromide (1 M in THF, 500 mL, 1.30 eq) was slowly added at 0°C. The mixture was stirred at 25°C for 4 hours. The reaction mixture was poured into a saturated NH₄Cl aqueous solution, and the organic layer and aqueous layer were separated. The aqueous layer was extracted with EtOAc (500 mL*3), and the organic layers were collected and concentrated in vacuo. The residue after concentration was purified using a silica column with petroleum ether:EtOAc = 50:1 → 10:1 to give 1-cyclopropylheptan-1-ol (46 g, 294.37 mmol, 76.4% yield) as a colorless oil.
¹H NMR (400 MHz, CHLOROFORM-d): *δ* 2.86 (td, 1H), 1.67 - 1.38 (m, 6H), 1.36 - 1.27 (m, 6H), 0.91 - 0.87 (m, 3H), 0.59 - 0.42 (m, 2H), 0.32 - 0.17 (m, 2H)

### (2) Synthesis of 1-cyclopropylheptyl 8-bromooctanoate

In a 2000 mL 3-neck RBF, 1-cyclopropylheptan-1-ol (10.0 g, 63.9 mmol, 1.00 eq) and 8-bromooctanoic acid (12.5 g, 63.9 mmol, 1.00 eq) in DCM (100 mL) were added. Then, DMAP (10.2 g, 83.19 mmol, 1.30 eq) and EDCI (15.9 g, 83.2 mmol, 1.30 eq) were added to the mixture. The mixture was stirred at 25°C for 16 hours. The reaction mixture was poured into H₂O (100 mL) and extracted, and then extracted again with DCM (100 mL*3). The organic layer was preserved and concentrated in vacuo. The residue after concentration was purified using a silica column with petroleum ether:EtOAc = 50:1 → 10:1 to obtain 1-cyclopropylheptyl 8-bromooctanoate (7.50 g, 22.5 mmol, 35.1% yield) as a colorless oil.
¹H NMR (400 MHz, CHLOROFORM-d): *δ* 4.28 (td, 1H), 3.59 - 3.36 (m, 2H), 2.35 - 2.26 (m, 2H), 1.92 - 1.72 (m, 2H), 1.69 - 1.60 (m, 4H), 1.50 - 1.41 (m, 2H), 1.37 - 1.24 (m, 12H), 1.01 - 0.84 (m, 4H), 0.62 - 0.50 (m, 1H), 0.50 - 0.41 (m, 1H), 0.37 (td, 1H), 0.26 (qd, 1H)

### (3) Synthesis of the compound of formula G

In a 500 mL 3-neck RBF, MeNH₂ (2 M in THF, 200.76 mL, 48.4 eq) was placed, and 1-cyclopropylheptyl 8-bromooctanoate (3.00 g, 8.30 mmol, 1.00 eq) was added to the flask under a nitrogen environment. The mixture was stirred at 80°C for 16 hours, and then the solvent was evaporated. The residue after evaporation was purified using a silica column with petroleum ether:EtOAc = 50:1 → 10:1 to obtain the compound of formula G (0.50 g, 844 µmol, 10.2% yield) as a yellow oil.
¹H NMR (400 MHz, CHLOROFORM-*d*): *δ* 0.26 (dq, 2 H) 0.34 - 0.40 (m, 2 H) 0.42 - 0.49 (m, 2 H) 0.50 - 0.58 (m, 2 H) 0.89 (br t, 6 H) 0.93 - 0.98 (m, 2 H) 1.26 - 1.34 (m, 26 H) 1.47 (br s, 4 H) 1.57 - 1.70 (m, 10 H) 2.13 - 2.41 (m, 11 H) 4.27 (dt, 2 H)

### Example 8

The compound of the following formula H was prepared according to the synthesis scheme shown in Figure 8. The synthesis method was the same as in Example 5 above, but 'cyclopropanecarbaldehyde' was replaced with 'cyclopentanecarbaldehyde,' and the same molar equivalents were used. 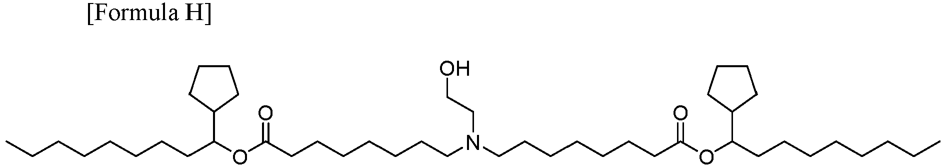 ¹H NMR (400 MHz, CHLOROFORM-d): *δ* = 4.26 (td, 2H), 3.54 (t, 2H), 2.55 (t, 2H), 2.50 - 2.41 (m, 4H), 2.28 (t, 4H), 1.70 - 1.56 (m, 10H), 1.49 - 1.39 (m, 4H), 1.35 - 1.21 (m, 49H), 0.95 (dt, 2H), 0.89 (t, 6H)

### Example 9

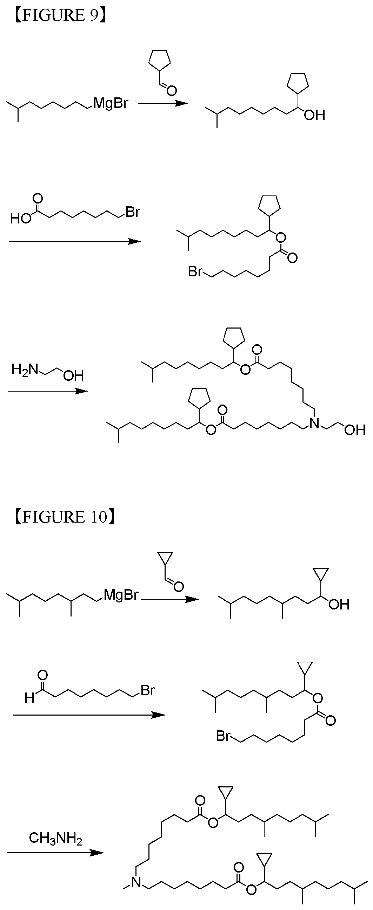

The compound of the following formula I was prepared according to the synthesis scheme shown in Figure 9. The synthesis method was the same as in Example 5 above, but 'cyclopropanecarbaldehyde' was replaced with 'cyclopentanecarbaldehyde' and 'octylmagnesium bromide' was replaced with '7-methyloctylmagnesium bromide,' and the same molar equivalents were used. 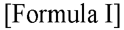 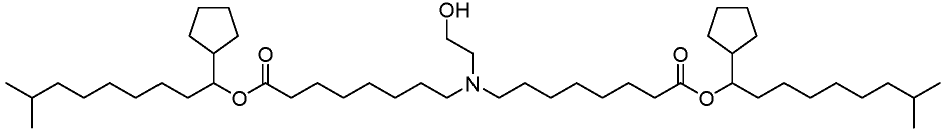 ¹H NMR (400 MHz, CHLOROFORM-*d*): *δ* = 4.28 (td, 2H), 3.50 (t, 2H), 2.58 (t, 2H), 2.49 - 2.40 (m, 4H), 2.30 (t, 4H), 1.71 - 1.60 (m, 10H), 1.50 - 1.41 (m, 4H), 1.35 - 1.20 (m, 49H), 0.97 (dt, 2H), 0.92 (t, 6H)

### Example 10

The compound of the following formula J was prepared according to the synthesis scheme shown in Figure 10. The synthesis method was the same as in Example 7 above, but 'hexylmagnesium bromide' was replaced with '(3,7-dimethyloxyl)magnesium bromide,' and the same molar equivalents were used. 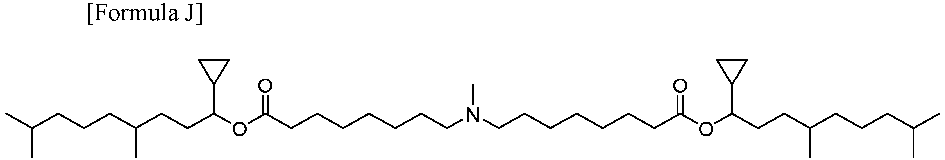 ¹H NMR (400 MHz, CHLOROFORM-d): 4.19 (dt, 2 H), 2.41 - 2.15 (m, 11 H), 1.71 - 1.51 (m, 10 H), 1.47 (br s, 4 H), 1.33 - 1.25 (m, 22 H), 0.98 - 0.93 (m, 2 H), 0.91 - 0.88 (m, 18H), 0.58 - 0.50 (m, 2 H), 0.49 - 0.42 (m, 2 H), 0.40 - 0.34 (m, 2 H), *δ* 0.26 (dq, 2 H)

### Example 11

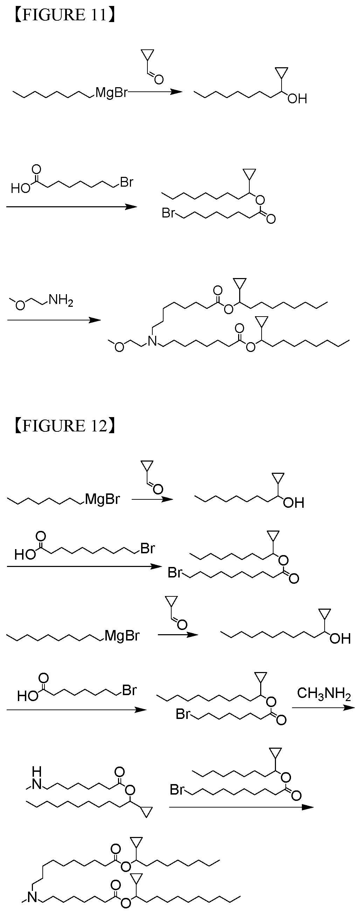

The compound of the following formula K was prepared according to the synthesis scheme shown in Figure 11. The synthesis method was the same as in Example 5 above, but '2-aminoethanol' was replaced with '2-methoxyethan-1-amine,' and the same molar equivalents were used. 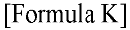 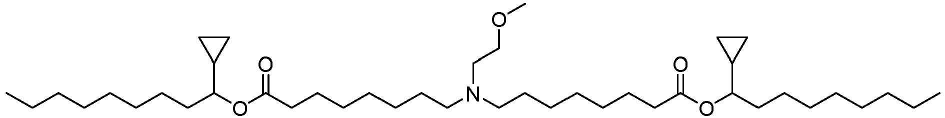 ¹H NMR (400 MHz, CHLOROFORM-*d*): *δ* = 4.25 (td, 2H), 3.69 (t, 2H), 3.35 (s, 3H), 2.60 (t, 2H), 2.45 - 2.39 (m, 4H), 2.38 (t, 4H), 1.72 - 1.57 (m, 10H), 1.43 - 1.34 (m, 4H), 1.30 - 1.25 (m, 33H), 0.95 (dt, 2H), 0.88 (t, 6H), 0.60 - 0.42 (m, 4H), 0.41 - 0.22 (m, 4H)

### Example 12

The compound of the following formula L was prepared according to the synthesis scheme shown in Figure 12. The synthetic method was similar to Example 3 above. '1-cyclopropylundecyl 8-bromooctanoate' and `1-cyclopropylnonyl 10-bromodecanoate' were each prepared, and then reacted with 'methylamine' to prepare a compound of formula L. 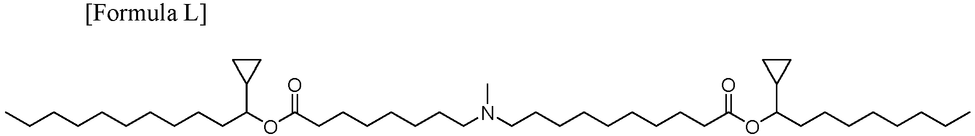 ¹H NMR (400 MHz, CHLOROFORM-*d*): *δ* 4.28 (td, 2H), 2.41 (br t, 8H), 2.22 (s, 3H), 1.80 - 1.66 (m, 8H), 1.50 - 1.39 (m, 4H), 1.39 - 1.23 (m, 48H), 1.02 (m, 2H), 0.87 (m, 6H), 0.61 - 0.22 (m, 8H)

### Example 13

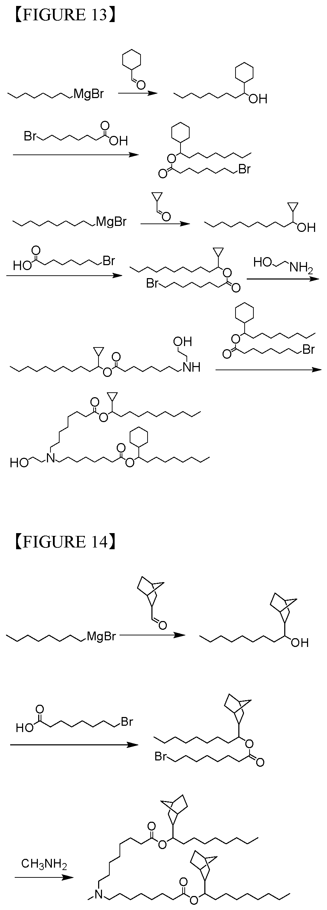

The compound of the following formula M was prepared according to the synthesis scheme shown in Figure 13. The synthetic method was similar to Example 5 above. '1-cyclopropylundecyl 8-bromooctanoate' and ` 1-cyclohexylnonyl 8-bromooctanoate' were each prepared, and then reacted with '2-aminoethanol' to prepare a compound of formula M. 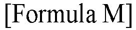 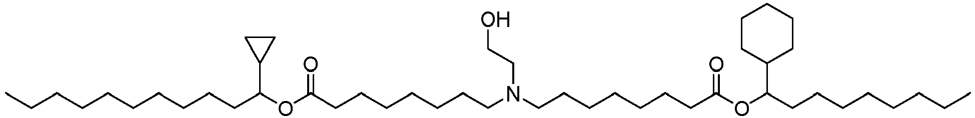 ¹H NMR (400 MHz, CHLOROFORM-*d*): *δ* = 4.30-4.27 (m, 2H), 3.52 (t, 2H), 2.57 (t, 2H), 2.47 - 2.41 (m, 4H), 2.31-2.29 (m, 4H), 1.70 - 1.57 (m, 8H), 1.48 - 1.41 (m, 4H), 1.36 - 1.26 (m, 57H), 0.94 (dt, 1H), 0.88 (t, 6H), 0.60 - 0.42 (m, 2H), 0.41 - 0.22 (m, 2H)

### Example 14

The compound of the following formula N was prepared according to the synthesis scheme shown in Figure 14. The synthesis method was the same as in Example 1 above, but 'cyclopropanecarbaldehyde' was replaced with 'bicyclo[2.2.1]heptane-2-carbaldehyde,' and the same molar equivalents were used. 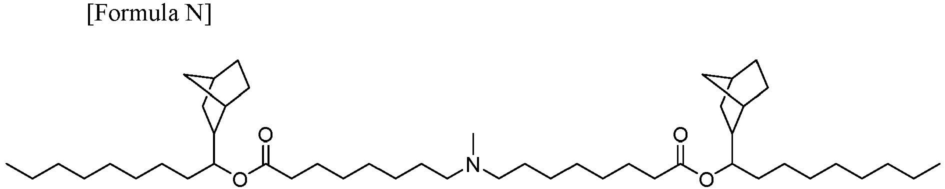 ¹HNMR (400 MHz, CHLOROFORM-*d*): *δ* 4.35 (td, 2H), 2.35-2.29 (m, 8H), 2.20 (s, 3H), 2.18 - 2.15 (m, 2H), 1.75 - 1.66 (m, 8H), 1.54 - 1.23 (m, 60H), 0.89 (t, 6H)

### Example 15

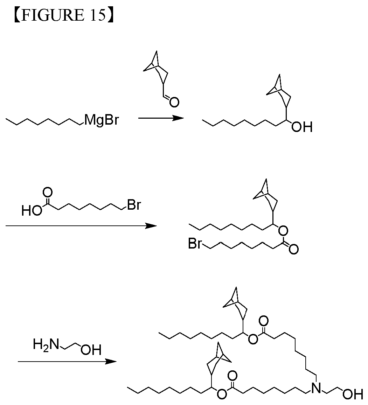

The compound of the following formula O was prepared according to the synthesis scheme shown in Figure 15. The synthesis method was the same as in Example 5 above, but 'cyclopropanecarbaldehyde' was replaced with 'bicyclo[3.1.1]heptane-3-carbaldehyde,' and the same molar equivalents were used. 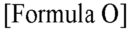 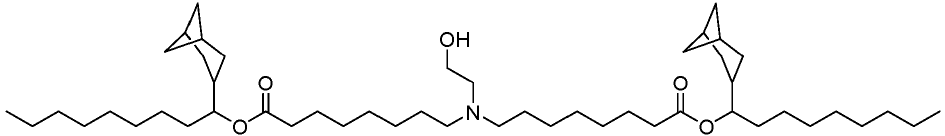 ¹H NMR (400 MHz, CHLOROFORM-*d*): *δ* = 4.40 (td, 2H), 3.54 (t, 2H), 2.56 (t, 2H), 2.47 - 2.40 (m, 4H), 2.30 (t, 4H), 2.22 (m, 2H), 1.71 - 1.58 (m, 10H), 1.48 - 1.26 (m, 54H), 0.99 - 0.79 (m, 10H)

### [Preparation Examples of composition for drug delivery: Preparation of formulations by using each compound of formulas A to G]

### 1. Raw material preparation

According to the following table 1, the materials required for preparing the formulation were dissolved in each dilution solvent and prepared at the required concentration. When dissolving, the materials were kept at room temperature and then the solvent was added to dissolve them.

**[Table 1]**

| | **Materials** | **Concentration for use** |
|---|---|---|
| 1 | mRNA | 1 mg/mL |
| 2 | Each compound of formulas A to G | 20 mg/mL |
| 3 | DOPE (dioleoyl-phosphatidyl-ethanolamine) | 10 mg/mL |
| 4 | Cholesterol | 10 mg/mL |
| 5 | DMG-PEG (1,2-dimyristoyl-rac-glycero-3-methoxypolyethylene glycol-2000) | 10 mg/mL |

### 2. Raw material mixing

The required amounts of the raw materials were mixed in order to meet the N/P ratio (amine group of lipid/phosphate group of mRNA) of 6 and each compound of formulas A to G:DOPE:Cholesterol:DMG-PEG=50:10:38.5:1.5. Ethanol was added to the ethanol layer so that the molecular total of all raw materials was within 12.5 mM, and the aqueous phase and ethanol phase were mixed maintaining a volume ratio of 3:1. After the mixing, to reduce the total ethanol content, buffer exchange was performed as follows: The mixture solution was concentrated by centrifugation at 4,000 rpm using an Amicon-Ultra tube filter (Merk Millipore, UFC505096 or UFC805024, pore size: 50K, volume: 15 mL), then diluted with PBS and centrifuged to concentrate. This process was repeated to exchange the buffer.

The more concrete procedure is as follows:
1) Two autoclaved tubes were prepared (tubes (A) and (B)).
2) In tube (A), each compound of formulas A to G, DOPE, cholesterol and DMG-PEG in molar quantities calculated according to the experimental conditions were sequentially added and mixed by vortexing.
3) In the ethanol phase, when necessary, ethanol was added so that the molecular total of all raw materials was within 12.5 mM.
4) In tube (B), mRNA and 20 mM sodium acetate buffer (pH 4.6) were mixed. At that time, the ratio was calculated and added so that the aqueous phase was total three times the amount of the ethanol phase.
5) Mixing of Tube (A) and Tube (B) was performed using a Microfluidics (Ignite, Precision Nanosystem) device. Microfluidics operating conditions were FRR (Flow Rate Ratio) of C:R=3:1 and TRR (Total Flow Rate) of 12 mL/min.
6) The resulting mixture from step 5) was centrifuged at 4,000 rpm using an Amicon-Ultra tube filter (50K), and the process of concentration and dilution was repeated to remove excess ethanol, and then concentrated to a final x mg/ml (theoretical concentration).

### 3. Evaluation of the properties of the formulation

1) For the prepared formulation, particle characteristics were confirmed using a particle size analyzer (Dynamic Light Scattering, DLS), and the results are shown in Table 2 below.
2) For the prepared formulation, mRNA encapsulation efficiency was confirmed through Ribo-green assay, and the results are shown in Table 1 below.

**[Table 2]**

| **Compound of formula** | **Z-average (nm)** | **PDI (PI)** | **Zeta-potential (mV)** | **Encapsulation efficiency (%)** |
|---|---|---|---|---|
| **A** | 135.0 ± 0.46 | 0.09 ± 0.01 | -10.96 ± 2.69 | 97.6 ± 0.3 |
| **B** | 183.3 ± 2.86 | 0.12 ± 0.03 | -4.30 ± 3.03 | 96.7 ± 0.3 |
| **C** | 120.0 ± 2.38 | 0.12 ± 0.00 | -7.57 ± 4.12 | 95.4 ± 0.2 |
| **D** | 154.6 ± 2.75 | 0.07 ± 0.04 | -2.37 ± 0.39 | 97.6 ± 0.2 |
| **E** | 127.4 ± 0.52 | 0.10 ± 0.04 | -4.69 ± 1.56 | 98.4 ± 0.5 |
| **F** | 13 1.5 ± 0.12 | 0.12 ± 0.02 | -6.24 ± 3.13 | 91.1 ± 0.2 |
| **G** | 194.9 ± 3.59 | 0.17 ± 0.02 | -6.93 ± 1.48 | 92.1 ± 0.1 |

## Claims

1. A a lipid having a structure represented by the following formula (1): 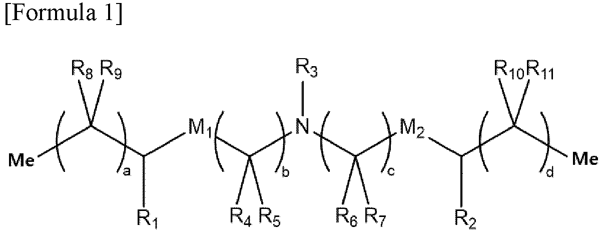 wherein, in the above formula (1),
each of M₁ and M₂ is independently a divalent linker group,
each of R₁ and R₂ is independently a substituted or unsubstituted carbocyclic group or heterocyclic group,
R₃ is hydrogen atom, or a substituted or unsubstituted organic group optionally comprising one or more heteroatoms,
each of R₄ to R₁₁ is independently hydrogen atom, or a substituted or unsubstituted, saturated or unsaturated hydrocarbon group,
Me is methyl group, and
each of a, b, c and d is independently an integer of from 1 to 20.

2. The lipid according to claim 1, wherein
each of M₁ and M₂ is independently selected from the group consisting of -C(O)O-, - OC(O)-, -OC(O)-M'-C(O)O-, -C(O)N(R')-, -N(R')C(O)-, -C(O)-, -C(S)-, -C(S)S-, - SC(S)-, -CH(OH)-, -P(O)(OR')O-, -S(O)₂-, -S-S-, arylene, and heteroarylene, wherein M' is a direct bond, C₁₋₁₃ alkylene or C₂₋₁₃ alkenylene, and each R' is independently selected from the group consisting of hydrogen atom, C₁₋₁₈ alkyl and C₂₋₁₈ alkenyl,
each of R₁ and R₂ is independently selected from the group consisting of substituted or unsubstituted C₃₋₂₀ cycloalkyl, substituted or unsubstituted C₃₋₂₀ cycloalkenyl, substituted or unsubstituted C₆₋₂₀ aryl, substituted or unsubstituted C₃₋₂₀ heterocycloalkyl, substituted or unsubstituted C₃₋₂₀ heterocycloalkenyl, and substituted or unsubstituted C₃₋₂₀ heteroaryl, R₃ is selected from the group consisting of hydrogen atom, substituted or unsubstituted C₁₋₆ alkyl, substituted or unsubstituted C₃₋₆ carbocyclic group, -(CH₂)ₙQ, - (CH₂)ₙCHQR, -CHQR and -CQ(R)₂, wherein each R is independently selected from the group consisting of hydrogen atom, C₁₋₃ alkyl and C₂₋₃ alkenyl; Q is selected from the group consisting of carbocyclic group, heterocyclic group, -OR, -O(CH₂)ₙN(R)₂, -C(O)OR, -OC(O)R, -CX₃, -CX₂H, -CXH₂, -CN, -N(R)₂, -C(O)N(R)₂, -N(R)C(O)R, -N(R)S(O)₂R, -N(R)C(O)N(R)₂, -N(R)C(S)N(R)₂, -N(R)R₁₂, N(R)S(O)2R₁₂, -O(CH2)ₙOR, -N(R)C(=NR₁₃)N(R)₂, - N(R)C(=CHR₁₃)N(R)₂, -OC(O)N(R)₂, -N(R)C(O)OR, -N(OR)C(O)R, -N(OR)S(O)₂R, - N(OR)C(O)OR, -N(OR)C(O)N(R)₂, -N(OR)C(S)N(R)₂, -N(OR)C(=NR₁₃)N(R)₂, - N(OR)C(=CHR₁₃)N(R)₂, -C(=NR₁₃)N(R)₂, - C(=NR₁₃)R, -C(O)N(R)OR and - C(R)N(R)₂C(O)OR, wherein each n is independently an integer of from 1 to 5; R₁₂ is selected from the group consisting of C₃₋₆ carbocyclic group and heterocyclic group; R₁₃ is selected from the group consisting of H, CN, NO₂, C₁₋₆ alkyl, -OR, -S(O)₂R, - S(O)₂N(R)₂, C₂₋₆ alkenyl, C₃₋₆ carbocyclic group and heterocyclic group; each R is independently selected from the group consisting of hydrogen atom, C₁₋₃ alkyl and C₂₋₃ alkenyl; each X is independently selected from the group consisting of F, CI, Br and I**,** provided that when R₃ is -(CH₂)ₙQ, -(CH₂)ₙCHQR, -CHQR or -CQ(R)₂, (i) if n is 1, 2, 3, 4, or 5, then Q is not -N(R)₂, or (ii) if n is 1 or 2, Q is not 5-, 6- or 7-membered heterocycloalkyl,
each of R₄ to R₁₁ is independently selected from the group consisting of hydrogen atom,
C₁₋₃ alkyl and C₂₋₃ alkenyl, and
each of a, b, c and d is independently an integer of from 1 to 15.

3. The lipid according to claim 1, wherein
each of M₁ and M₂ is independently selected from the group consisting of -C(O)O-, - OC(O)-, -OC(O)-M'-C(O)O-, -C(O)N(R')-, -N(R')C(O)- and -C(O)-, wherein M' and R' are the same as defined in Claim 2,
each of R₁ and R₂ is independently selected from the group consisting of substituted or unsubstituted C₃₋₂₀ cycloalkyl and substituted or unsubstituted C₃₋₂₀ heterocycloalkyl,
R₃ is selected from the group consisting of hydrogen atom, substituted or unsubstituted C₁₋₆ alkyl, and substituted or unsubstituted C₃₋₆ carbocyclic group,
each of R₄ to R₁₁ is independently hydrogen atom or C₁₋₃ alkyl, and
each of a, b, c and d is independently an integer of from 3 to 11.

4. The lipid according to claim 1, wherein
each of M₁ and M₂ is independently -C(O)O- or -OC(O)-,
each of R₁ and R₂ is independently substituted or unsubstituted C₃₋₆ cycloalkyl,
R₃ is hydrogen atom or unsubstituted C₁₋₃ alkyl,
R₄ to R₁₁ are hydrogen atom, and
each of a, b, c and d is independently an integer of from 5 to 9.

5. The lipid according to claim 1, wherein the lipid is one having a structure selected from the following formulas A to O:
| Formula | Structure |
|---|---|
| A | 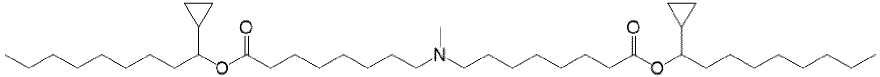 |
| B | 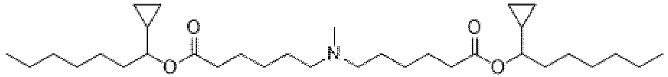 |
| C | 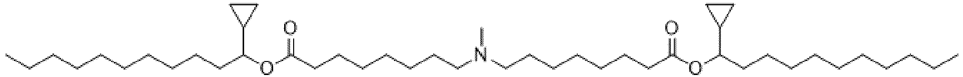 |
| D | 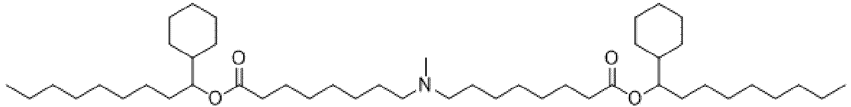 |
| E | 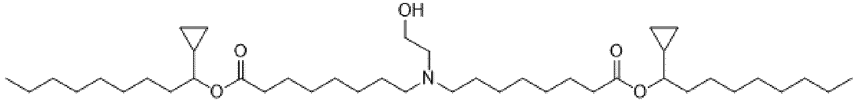 |
| F | 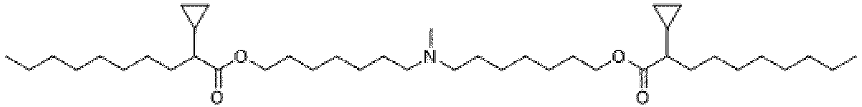 |
| G | 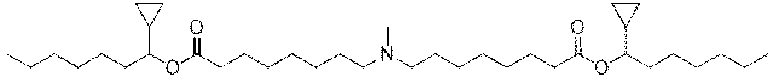 |
| H | 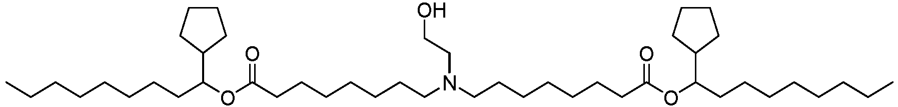 |
| I | 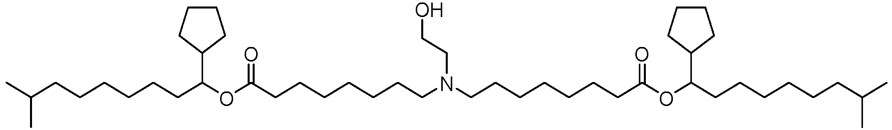 |
| J | 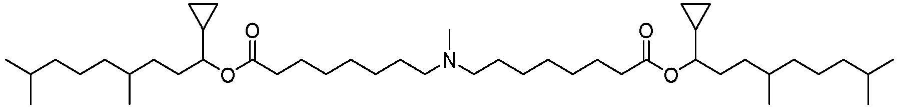 |
| K | 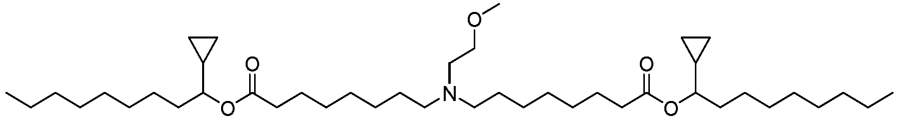 |
| L | 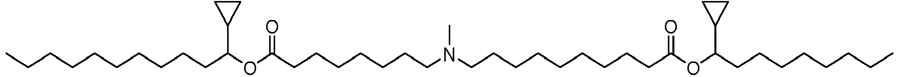 |
| M | 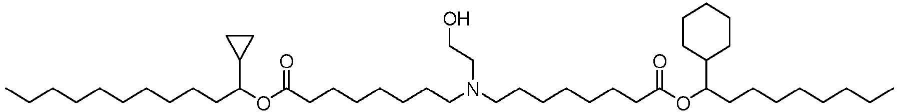 |
| N | 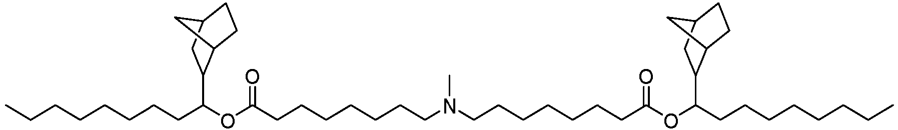 |
| O | 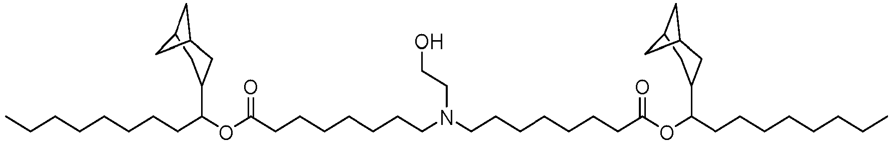 |

6. A method for preparing a lipid having a structure represented by formula (1'), comprising steps of:
(1) reacting a compound of formula (a) with a compound of formula (b) to obtain a compound of formula (c);
(2) reacting a compound of formula (c) with a compound of formula (d) to obtain a compound of formula (e); and
(3) reacting a compound of formula (e) with a compound of formula (f): 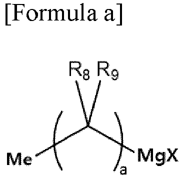
[Formula b] R₁-CHO 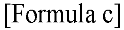 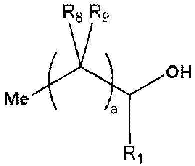 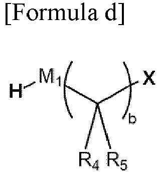 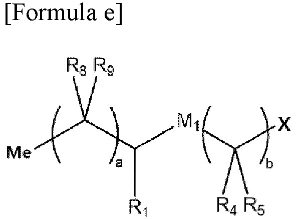
[Formula f] R₃-NH₂ 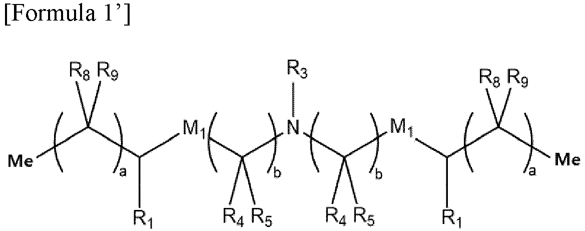
wherein,
M₁, R₁, R₃, R₄, R₅, R₈, R₉, Me, a and b are the same as defined in claim 1, and
each X is independently selected from the group consisting of F, CI, Br and I.

7. A method for preparing a lipid having a structure represented by formula (1), comprising steps of:
(1) reacting a compound of formula (a') with a compound of formula (b') to obtain a compound of formula (c');
(2) reacting a compound of formula (c') with a compound of formula (d') to obtain a compound of formula (e'); and
(3) reacting a compound of formula (e') and a compound of formula (e) obtained in claim 6 with a compound of formula (f): 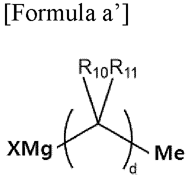
[Formula b'] R₂-CHO 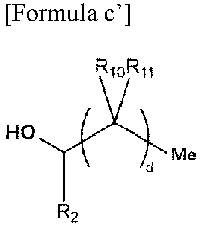 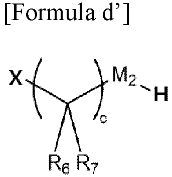 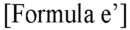 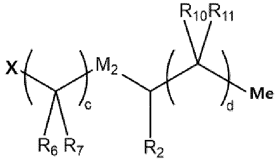 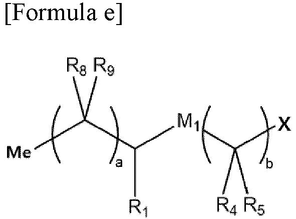
[Formula f] R₃-NH₂ 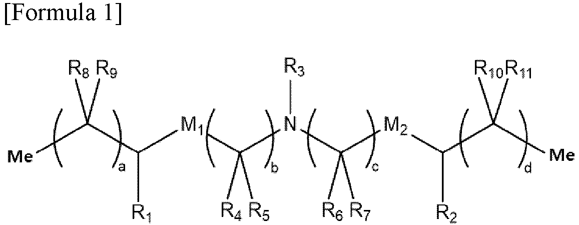
wherein,
M₁, M₂, R₁ to R₁₁, Me, a, b, c and d are the same as defined in claim 1, and
each X is independently selected from the group consisting of F, CI, Br and I.

8. A method for preparing a lipid having a structure represented by formula (1'), comprising steps of:
(1) reacting a compound of formula (i) with a compound of formula (ii) to obtain a compound of formula (iii);
(2) reacting a compound of formula (iii) with a compound of formula (iv) to obtain a compound of formula (v);
(3) reacting a compound of formula (v) with a compound of formula (vi) to obtain a compound of formula (vii); and
(4) reacting a compound of formula (v) with a compound of formula (vii): 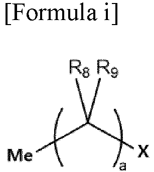 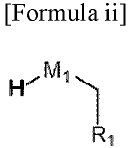 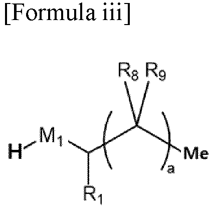 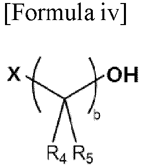 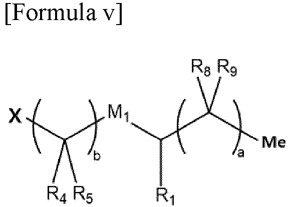
[Formula vi] R₃-NH₂ 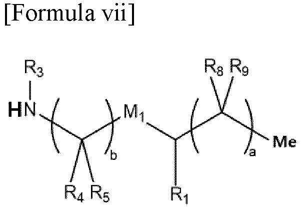 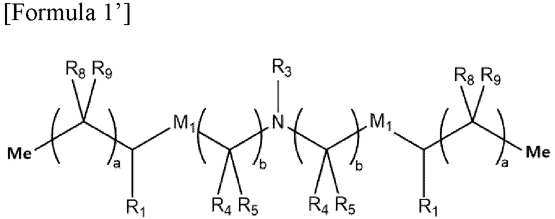
wherein,
M₁, R₁, R₃, R₄, R₅, R₈, R₉, Me, a and b are the same as defined in claim 1, and
each X is independently selected from the group consisting of F, CI, Br and I.

9. A method for preparing a lipid having a structure represented by formula (1), comprising steps of:
(1) reacting a compound of formula (i') with a compound of formula (ii') to obtain a compound of formula (iii');
(2) reacting a compound of formula (iii') with a compound of formula (iv') to obtain a compound of formula (v');
(3) reacting a compound of formula (v') with a compound of formula (vi) to obtain a compound of formula (vii'); and
(4) reacting a compound of formula (vii') with a compound of formula (v) obtained in claim 8: 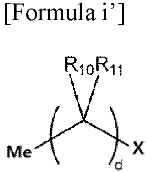 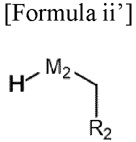 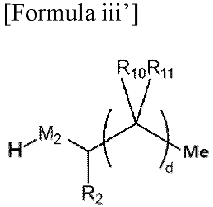 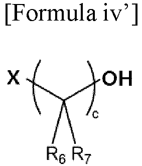 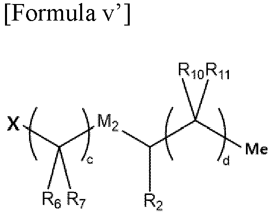
[Formula vi] R₃-NH₂ 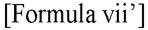 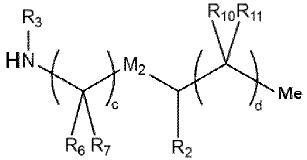 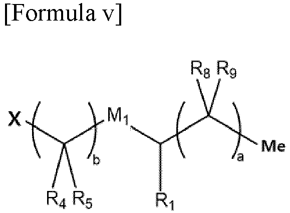 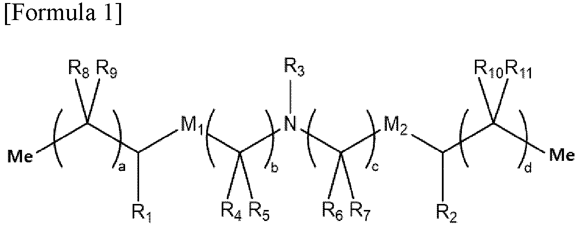
wherein,
M₁, M₂, R₁ to R₁₁, Me, a, b, c and d are the same as defined in claim 1, and
each X is independently selected from the group consisting of F, CI, Br and I.

10. A composition for drug delivery comprising the lipid of any one of claims 1 to 5.
